# EUROPEAN PATENT APPLICATION

(11) **EP 2 186 814 A1**
(43) Date of publication of application: **19.05.2010**
(21) Application number: 08783626.8
(22) Date of filing: 07.08.2008
(51) Int. Cl.: C07D 477/20, A61K 31/40, A61P 31/00

(54) **SULFONYL-SUBSTITUTED CARBAPENEM COMPOUNDS**

(30) Priority: 09.08.2007 CN 200710016461
(71) Applicant: KBP Biosciences Co., Ltd., National High-Tech Development Zone Jinan, Shandong 250-101 (CN)
(72) Inventor: HUANG, Zhenhua, Shandong 250101 (CN); SUN, Liang, Jinan, Shandong 250101 (CN)
(74) Representative: Häckel, Stefan
(86) International application number: PCT/CN2008/001440
(87) International publication number: WO 2009/018723

(57) **Abstract**

The present invention relates to a compound of the formula (I), pharmaceutically acceptable salts, hydrolysable esters, isomers and hydrates thereof, and hydrates of the said esters or salts, wherein R¹, R², R³ and 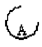 are as defined in the description. The present invention further relates to a process for preparing the compounds, to pharmaceutical compositions comprising the compounds, and to the use of the compounds in the manufacture of a medicament for the treatment and/or prophylaxis of infectious diseases.

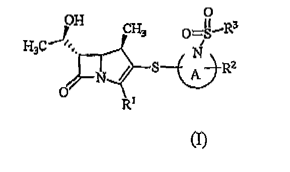

## Description

### Technical Field

The present application relates to sulfonyl-substituted carbapenems, the pharmaceutically acceptable salts, the hydrolysable esters, the isomers and the hydrates thereof, and hydrates of the said esters or salts, to the processes for preparing the same, to the pharmaceutical compositions containing such compounds, and to the use of these compounds in the manufacture of a medicament for the treatment and/or prophylaxis of infectious diseases.

### Background Art

Carbapenem antibiotics attract a lot of attention for their broad antibacterial spectrum, potent antibacterial activity, and stability to β-lactamase. Carbapenem antibiotics that have been clinically used include imipenem, meropenem, panipenem, biapenem, and doripenem and the like. Doripenem is a carbapenem antibiotic developed by the Shionogi & Co. Ltd., Japan, and has the following structure:

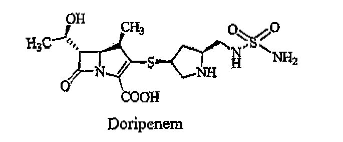

Doripenem has a better antibacterial action against Gram positive and negative bacteria than that of imipenem, and has antibacterial action against imipenem-resistant bacteria. Having a good stability to renal dehydropeptidase-I (DHP-1) containing serine sites, doripenem is mainly used for the treatment of severe infection in urinary system and respiratory system. However, due to too much clinical application of doripenem, bacteria have increased resistance to doripenem.

Meropenem, which was firstly commercialized in Italy in 1995, is a carbapenem antibiotic developed by the Sumitomo Pharmaceuticals Co. Ltd, Japan. It has the following structure:

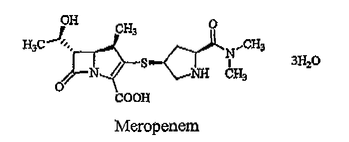

Meropenem is the first commercialized 1β-methyl carbapenem antibiotic. Due to more and more clinical application of meropenem, bacteria gradually develop resistance to meropenem.

Due to the increase of drug-resistance of bacteria caused by the abuse of antibiotics, and to the limitation of absorption in alimentary track, the currently marketed carbapenems are administered only as injections in clinic, and thus their clinical availability is limited, In addition, both meropenem and doripenem have short half-livies of about 1h in human body, which cannot satisfy the clinical needs.

In view of the challenge of drug-resistance of bacterial against the currently marketed meropenem and doripenem, there is a dire need to find new carbapenems exhibiting antibacterial action against Gram positive and negative bacteria higher than or equivalent to those of meropenem and doripenem, and having resistance to DHP-I. Moreover, such new carbapenems shall have a longer half-life, so as to satisfy the clinical needs.

### Summary of Invention

The present invention provides the following technical solutions:
[1]. A compound of the formula (I), pharmaceutically acceptable salts, hydrolysable esters, isomers and hydrates thereof, or hydrates of the said esters or salts: 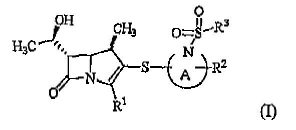 wherein
   R¹ represents a carboxyl group, -COOR⁴ or a hydrolysable ester, said R⁴ representing a carboxyl protecting group; 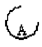 represents a saturated or unsaturated 3- to 7-membered heterocycle containing 1 to 2 nitrogen atoms;
   R² represents a hydrogen atom, a halogen atom, hydroxy, amino, carboxy, cyano, nitro, trifluoromethyl, a lower alkyl group, or a lower alkoxy group; and
   R³ represents hydroxy or -NR⁵R⁶, where each of R⁵ and R⁶ independently represents a hydrogen atom or a lower alkyl group, said lower alkyl group being optionally substituted with one or more substituents selected from the group consisting of hydroxy, amino, amido, aminosulfonyl, a halogen atom, carboxy, cyano, a lower alkoxy group, trifluoromethoxy, difluoromethoxy trifluoromethyl, and a combination thereof.
[2]. A compound according to t[1], wherein the compound of the formula (I) has the formula (I'): 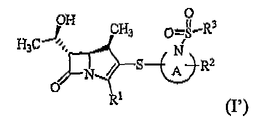 wherein
   R¹ represents a carboxyl group, -COOR⁴ or a hydrolysable ester, said R⁴ representing a carboxyl protecting group;
   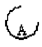 represents a saturated or unsaturated 3- to 7-membered heterocycle containing 1 to 2 nitrogen atoms;
   R² represents a hydrogen atom, a halogen atom, hydroxy, amino, carboxy, cyano, nitro, trifluoromethyl, a lower alkyl group, or a lower alkoxy group; and
   R³ represents hydroxy or -NR⁵R⁶, Where each of R⁵ and R⁶ independently represents a hydrogen atom or a lower alkyl group, said lower alkyl group being optionally substituted with one or more substituents selected from the group consisting of hydroxy, amino, amido, aminosulfonyl, a halogen atom, carboxy, cyano, a lower alkoxy group, trifluoromethoxy, difluoromethoxy, trifluoromethyl, and a combination thereof.
[3]. A compound according to [1], pharmaceutically acceptable salts, hydrolysable esters, isomers and hydrates thereof, or hydrates of the said esters or salts, wherein R¹ represents a carboxyl group, -COOR⁴ or a hydrolysable ester, said R⁴ representing a carboxyl protecting group which is selected from the group consisting of methyl, methoxymethyl, methylthiomethyl, benzoxymethyl, benzoylmethyl, ethyl, tert-butyl, allyl, benzyl, and p-nitrobenzyl, and said hydrolysable ester being selected from the group consisting of lower alkanoyloxyalkyl esters, cycloalkanoyloxyalkyl esters, lower alkoxylacyloxyalkyl esters, cycloalkoxylacyloxyalkyl esters, and (5-methyl-2-oxo-1,3-diaxol-4-yl) methyl esters;
   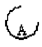 represents a saturated or unsaturated 4- to 6-membered heterocycle containing 1 to 2 nitrogen atoms, which is selected from the group consisting of azetidine, 1,2-diazetidine, 1,2-dihydroazetine, 1,2-dihydro-1,2-diazetine, pyrrolidine, pyrrole, dihydropyrrole, pyrazole, pyrazolidine, imidazole, azacyclohexane, 5,6-dihydropyrimidine, tetrahydropyrimidine, piperidine, and piperazine;
   R² represents a hydrogen atom, a fluorine atom, hydroxy, amino, carboxy, methyl, trifluoromethyl, ethyl, methoxy, or ethoxy; and
   R³ represents -NR⁵R⁶, where each of R⁵ and R⁶ independently represents a hydrogen atom or a substituted or unsubstituted C₁-C₆ alkyl.
[4]. A compound according to [3], pharmaceutically acceptable salts, hydrolysable esters, isomers or hydrates thereof, or hydrates of the said esters or salts, wherein
   R¹ represents a carboxyl group, -COOR⁴ or a hydrolysable ester, said R⁴ representing a carboxyl protecting group which is selected from the group consisting of methyl, methoxymethyl, methylthiomethyl, benzoxymethyl, benzoylmethyl, ethyl, tert-butyl, allyl, benzyl, and p-nitrobenzyl, and said hydrolysable ester being selected from the group consisting of lower alkanoyloxyalkyl esters, cycloalkanoyloxyalkyl esters, lower alkoxylacyloxyalkyl esters, cycloalkoxylacyloxyalkyl esters, and (5-methyl-2-oxo-1,3-dioxol-4-yl) methyl esters;
   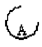 is selected from the group consisting of azetidine, pyrrolidine, piperidine, 5,6-dihydmpyrimidine, and tetrahydropyrimidine;
   R² represents a hydrogen atom; and
   R³ represents hydroxy or -NR⁵R⁶, where each of R⁵ and R⁶ independently represents a hydrogen atom or C₁-C₄ alkyl, said C₁-C₄ alkyl being optionally substituted with one or more substituents selected from the group consisting of hydroxy, amino, amido, and a combination thereof.
[5]. A compound according to [4], pharmaceutically acceptable salts, hydrolysable esters, isomers and hydrates thereof or hydrates of the said esters or salts, wherein
   R¹ represents a carboxyl group, -COOR⁴ or a hydrolysable ester, said R⁴ representing a carboxyl protecting group which is selected from the group consisting of methyl, allyl, and benzyl, and said hydrolysable ester being selected from the group consisting of propionyloxymethyl esters, butyryloxymethyl esters, tert-butylformyloxymethyl esters, isopropoxyformyloxymethyl esters, isopropoxyformyloxy-1-ethyl esters, cyclohexanoxyformyloxy-1-ethyl esters and (5-methyl-2-oxo-1,3-dioxol-4-yl) methyl esters;
   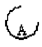 is selected from the group consisting of azetidine, pyrrolidine, piperidine, 5,6-dihydropyrimidine, and tetrahydropyrimidine;
   R² represents a hydrogen atom; and
   R³ represents -NH₂.
[6]. A compound according to [1], or pharmaceutically acceptable salts, hydrolysable esters, isomers and hydrates thereof, or hydrates of the said esters or salts, said compound being selected from the group consisting of:
   (4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxylic acid (compound 1);
   (4R,5S,6S)-3-[(35)-N-aminosulfonyl-pyrrolidin-3-yl]thlo-6-[(1R)-1-hydroxyethyl] -4-methyl-7-oxo-1-aza-bicyclo-[3.2.0]hept-2-ene-2-carboxylic acid (compound 2);
   (4R,5S,6S)-3-[N-aminosulfonyl-piperidin-3-yl]thio-6-[(1R)-1-hydroxyethyl]-4-methyl-7-oxo-1-aza-bicyclo-[3.2.0]hept-2-ene-2-carboxylic acid (compound 3);
   (4R,5S,6S)-3-[1(4*H*)-aminosulfonyl-5,6-dihydropyrimidin-5-yl]thio-6-[(1R)-1-hydrcxyethyl]-4-methyl-7-oxo-1-aza-bicyclo-[3.2.0]hept-2-ene-2-Carboxylic acid (compound 4);
   (4R,5S,6S)-3-[1(2*H*)-aminosulfonyl-tetrahydropyrimidin-5-yl]thio-6-[(1R)-1-hydroxyethyl]-4-methyl-7-oxo-1-aza-bicyclo-[3.2.0]hept-2-ene-2-carboxylic acid (compound 5);
   (4R,SS,6S)-3-[1-(N,N-dimethylaminosulfonyl)-azetidin-3-yl]thio-6-[(1R)-1-hydroxyethyl]-4-methyl-7-oxo-1-aza-bicyclo-[3.2.0]hept-2-ene-2-carboxylic acid (compound 6);
   (4R,5S,6S)-3-[1-(N,N-diethylaminosulfonyl)-azetidin-3-yl]thio-6-[(1R)-1-hydroxyethyl]-4-methyl-7-oxo-1-aza-bicyclo-[3.2.0]hept-2-ene-2-carboxylic acid (compound 7);
   (4R,5S,6S)-3-[1-(N,N-dibutylaminosulfonyl)-azetidin-3-yl]thio-6-[(1R)-1-hydroxyethyl]-4-methyl-7-oxo-1-aza-bicyclo-[3.2,0]hept-2-ene-2-carboxylic acid (compound 8);
   (4R,5S,6S)-3-[1-(2-hydroxy-3-aminopropylaminosulfonyl)-azetidin-3-yl]thio-6-[(1R)-1-hydroxyethyl]-4-methyl-7-oxo-1-aza-bicyclo-[3,2.0]hept-2-ene-2-carboxylic acid (compound 9); and
   (4R,5S,6S)-3-[1-(2-acetamido)aminosulfonyl-azetidin-3-yl]thio-6-[(1R)-1-hydroxyethyl]-4-methyl-7-oxo-1-aza-bicyclo-[3.2.0]hept-2-ene-2-carboxylic acid (compound 10).
[7]. A compound according to any one of [1] to [6], pharmaceutically acceptable salts thereof, hydrolysable esters thereof, isomers thereof, hydrates thereof, or hydrates of the said esters or salts, wherein the pharmaceutically acceptable salts are organic acid salts, inorganic acid salts, organic base or inorganic base salts, wherein said organic acids are selected from acetic acid, trifluoroacetic acid, methanesulfonic acid, toluenesulfonic acid, maleic acid, succinic acid, tartaric acid, citric acid, and fumaric acid; said inorganic acids are selected from hydrochloric acid, hydrobromic acid, nitric acid, sulphuric acid, and phosphoric acid; said organic bases are selected from meglumine and dextrosamine: and said inorganic bases are selected from the alkaline compounds containing sodium, potassium, barium, calcium, magnesium, zinc or lithium.
[8]. A compound according to any one of [1] to [7], wherein the hydrolysable esters are those that can be hydrolyzed into the corresponding carboxylic acids in a biological body.
[9]. A pharmaceutical composition which comprises a compound according to any one of [1] to [8], pharmaceutically acceptable salts, hydrolysable esters, isomers or hydrates thereof, or hydrates of the said esters or salts, and one or more pharmaceutically acceptable carriers and/or diluents.
[10]. A pharmaceutical composition according to [9], which is in any pharmaceutically acceptable dosage form.
[11]. Use of a compound according to any one of [1] to [8], pharmaceutically acceptable salts, hydrolysable esters, isomers or hydrates thereof, or hydrates of the said esters or salts in the manufacture of a medicament for the treatment and/or prophylaxis of infectious diseases.
[12]. A process for preparing a compound of the formula (I), which comprises performing a nucleophilic substitution reaction between a compound of the formula (II) or an isomer /ester/salt thereof with a compound of formula (III), 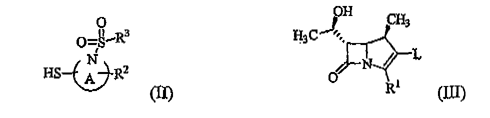 wherein R¹, R², R³ and 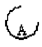 are as defined in the technical solution [1], and L represents a leaving group.
[13]. A compound of the formula (II), or a salt, hydrolysable ester or isomer thereof, 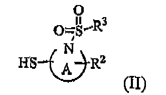 wherein R², R³ and 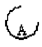 are as defined in the technical solution [1].

### Detailed Description of the Invention

The present invention provides a compound of the formula (I), pharmaceutically acceptable salts, hydrolysable esters, isomers and hydrates thereof, or hydrates of the said esters or salts: 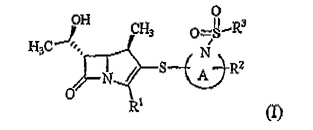 wherein
R¹ represents a carboxyl group, -COOR⁴ or a hydrolysable ester, said R⁴ representing a carboxyl protecting group;
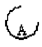 represents a saturated or unsaturated 3- to 7-membered heterocycle containing 1 to 2 nitrogen atoms;
R² represents a hydrogen atom, a halogen atom, hydroxy, amino, carboxy, cyano, nitro, trifluoromethyl, a lower alkyl group, or a lower alkoxy group; and R³ represents hydroxy or -NR⁵R⁶, where each of R⁵ and R⁶ independently represents a hydrogen atom or a lower alkyl group, said lower alkyl group being optionally substituted with one or more substituents selected from the group consisting of hydroxy, amino, amido, aminosulfonyl, a halogen atom, carboxy, cyano, a lower alkoxy group, trifluoromethoxy, difluoromethoxy trifluoromethyl, and a combination thereof.

The term "lower alkyl group" as used herein, means a straight or branch chain alkyl group containing 1 to 6 carbon atoms, such as methyl, ethyl, propyl, isopropyl, butyl, isobutyl, tert-butyl, sec-butyl, pentyl, neopentyl, hexyl, or analogs thereof.

The term "lower alkoxy group" as used herein, means a straight or branch chain alkoxy group containing 1 to 6 carbon atoms, such as methoxy, ethoxy, propoxy, isopropoxy, butoxy, isobutoxy, tert-butoxy, sec-butoxy, pentoxy, neopentoxy, hexyloxy, or analogs thereof.

The aforesaid lower alkyl group and lower alkoxy group are optionally substituted with one or more substituents selected from the group consisting of hydroxy, amino, amido, aminosulfonyl, a halogen atom, carboxy, cyano, a lower alkoxy group, trifluoromethoxy, difluoromethoxy, trifluoromethyl, and a combination thereof. Said "amido" includes aminoformyl, aminoacetyl, aminopropanoyl, aminobutyryl, and etc.

The term "3- to 7-membered heterocycle containing 1 to 2 nitrogen atoms" as used herein, means a saturated or unsaturated 3- to 7-membered heterocycle containing 1 to 2 nitrogen atoms, such as aziridine, diaziridine, azetidine, 1,2-diazetidine, 1,2-dihydroazetine, 1,2-dihydto-1,2-diazetine, pyrrolidine, pyrrole, dihydropyrrole, pyrazole, pyrazolidine, imidazole, azacyclohexane, 5,6-dihydropyrimidine, tetrahydropyrimidine, piperidine, piperazine, azacycloheptane, and analogs thereof.

The term "carboxyl-protecting group" as used herein, means a protecting group which can be conventionally used to substitute the acidic proton of the carboxyl group. Examples of such group include methyl, methoxymethyl, methylthiomethyl, tetrahydropyran, tetrahydrofuranyl, methoxyethylmethyl, allyl, benzyloxymethyl, phenacyl, p-bromophenacyl, α-methylphenacyl, p-methoxyphenacyl, diacylmethyl, N-phthalimidomethyl, ethyl, 2,2,2-trichloroethyl, 2-haloethyl, ω-chloroalkyl, 2-(trimethylsilyl)ethyl, 2-methylthioethyl, 2-(p-nitrophenylthio)ethyl, 2-(p-methylphenylthio)ethyl, 1-mothyl-1-phenylethyl, tert-butyl, cyclopentyl, cyclohexyl, di-(o-nitrophenyl)methyl, 9-fluorenylmethyl, 2-(9,10-dioxo)-fluorenylmethyl, 5-diphenylthio, benzyl, 2,4,6-trimethylbenzyl, p-bromobenzyl, o-nitrobenzyl, p-nitrobenzyl, p-methoxybenzyl, piperonyl, 4-pyridinylmethyl, trimethylsilyl, triethylsilyl, tert-butyldimethylsilyl, isopropyldimethylsilyl, phenyldimethylsilyl, S-tert-butyl, S-phenyl, S-2-pyridyl, N-hydroxypiperidinyl, N-hydroxysuccinimido, N-hydroxyphthalimido, N-hydroxybenzotriazolyl, O-acyloxime, 2,4-dinitrophenylthio, 2-alkyl-1,3-oxazoline, 4-alkyl-5-oxo-1,3-oxazolidine, 5-alkyl-4-oxo-1,3-dioxane, triethylstannane, tri-n-butylstannane, N,N'-diisopropylhydrazide, or analogs thereof.

Tn the present invention, the following compounds are preferred:
Chemical name: (4R,SS,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxylic acid, hereafter referred as compound 1, which has the following structure: 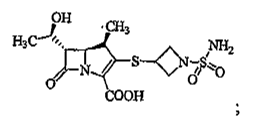
Chemical name: (4R,5S,6S)-3-[(3S)-N-aminosulfonyl-pyrrolidin-3-yl]thio-6-[(1R)-1-hydroxyethyl]-4-methyl-7-oxo-1-aza-bicyclo-[3.2.0]hept-2-ene-2-carboxylic acid, hereafter referred as compound 2, which has the following structure: 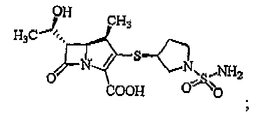
Chemical name: (4R,5S,6S)-3-[N-aminosulfonyl-piperidin-3-yl]thio-6-[(1R)-1-hydroxyethyl]-4-methyl-7-oxo-1-aza-bicyclo-[3.2.0]hept-2-ene-2-carboxylic acid, hereafter referred as compound 3, which has the following structure: 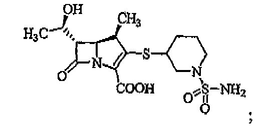
Chemical name: (4R,5S,6S)-3-[1(4*H*)-aminosulfonyl-5,6-dihydropyrimidin-5-yl]-thio-6-[(1R)-1-hydroxyethyl]-4-methyl-7-oxo-1-aza-bicyclo-[3.2.0]hept-2-ene-2 carboxylic acid, hereafter referred as compound 4, which has the following structure: 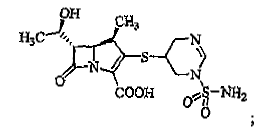
Chemical name: (4R,5S,6S)-3-[1(2*H*)-aminosulfonyl-tetrahydropyrimidin-5-yl]-thio-6-[(1R)-1-hydroxyethyl]-4-methyl-7-oxo-1-aza-bicyclo-[3.2.0]hept-2-ene-2-carboxylic acid, hereafter referred as compound 5, which has the following structure: 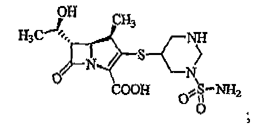
Chemical name: (4R,5S,6S)-3-[1-(N,N-dimethylaminosulfonyl)-azetidin-3-yl]-thio-6-[(1R)-1-hydroxyethyl]-4-mehyl-7-oxo-1-aza-bicyclo-[3.2.0]hept-2-ene-2, carboxylic acid, hereafter referred as compound 6, which has the following structure; 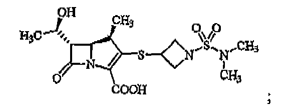
Chemical name: (4R,5S,6S)-3-[1-(N,N-diethylaminosulfonyl)-azetidin-3-yl]thio-6-[(1R)-1-hydroxyethyl]-4-methyl-7-oxo-1-aza-bicyclo-[3.2.0]hept-2-ene-2-carboxylic acid, hereafter referred as compound 7, which has the following structure: 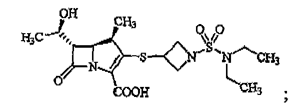
Chemical name; (4R,5S,6S)-3-[1-(N,N-dibutylaminosulfonyl)-azetidin-3-yl]thio-6-[(1R)-1-hydroxyethyl]-4-methyl-7-oxo-1-aza-bicyclo-[3.2.0]hept-2-ene-2-carboxylic acid, hereafter referred as compound 8, which has the following structure: 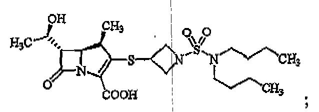
Chemical name: (4R,5S,6S)-3-[1-(2-hydroxy-3-aminopropylaminosulfonyl)-azetidin-3-yl]thio-6-[(1R)-1-hydroxyethyl]4-methyl-7-oxo-1-aza-bicyclo-[3.2.0]-hept-2-ene-2-carboxylic acid, hereafter referred as compound 9, which has the following structure: 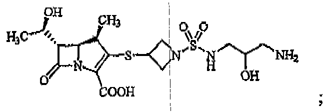 and
Chemical name: (4R,5S,6S)-3-[1-(2-acetamido)-aminosulfonyl)azetidin-3-yl]-thio-6-[(1R)-1-hydroxyethyl]-4-methyl-7-oxo-1-aza-bicyclo-[3.2.0]hept-2-ene-2-carboxylic acid, hereafter referred as compound 10, which has the following structure:

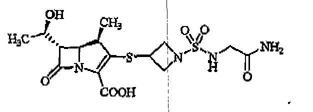

In addition, the present invention provides a process for preparing a compound of the formula (I), which comprises performing a nucleophilic substitution reaction between a compound of the formula (II) or a salt/ester/isomer thereof and a compound of formula (III), 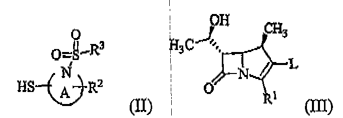 wherein R², R³ and 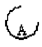 in formula (II) are as defined above.

In formula (III), R¹ is as defined above, and L represents a leaving group. The "leaving group" includes, for example, the reactive group of a hydroxy group, such as sulphonate (e.g. lower alkanesulfonyloxy, trifluoromethanesulfonyloxy, benzenesulphonyloxy, toluenesulphonyloxy), phosphates (e.g. diarylphosphate, such as, diphenylphosphate) or halides (e.g. chlorides); sulphoxide, such as -SOCH=CH-NHCOCH₃, which can be readily replaced. The preferred L is diphenylphosphate (-OP(O)(OPh)₂).

The present invention further provides a process for preparing the aforesaid compounds as illustrated, but not limited to, the following reaction scheme:

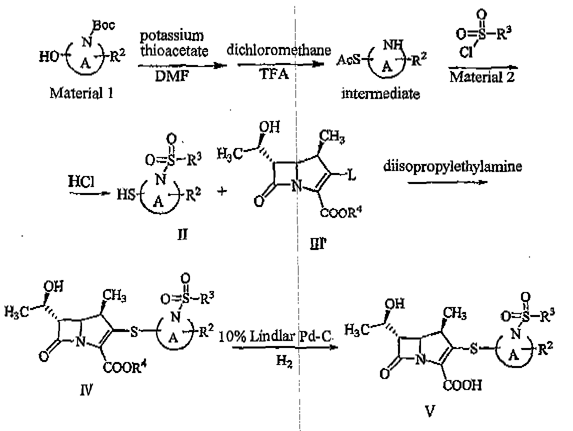

### Reaction steps;

### Step 1 Preparation of the compound of the formula (II)

To a dried flask, Material 1 and potassium thioacetate were added, and then DMF was added thereto. The mixture was heated with stirring until dissolution. The mixture was kept warm for 2 to 20 hours. The resultant mixture was cooled to ambient temperature, and water was added to dilute the mixture. The resultant was extracted with ethyl acetate. The organic phase was washed with water from one to three times, and then dried and concentrated to give an oil. Dichloromethane was added to the obtained oil, and, at ambient temperature, trifluoroacetic acid (TFA) was added thereto dropwise. The obtained mixture was stirred over night. TLC was used to monitor whether the reactants therein had been fully reacted. The resultant was concentrated, dichloromethane was added thereto to dilute the concentrate, and the resultant was rotary-evaporated to dryness to give the residue. The above operations were repeated for 1 to 5 times to remove the residual TFA. To the resultant mixture, methanol was added, so that a solid, i.e. the intermediate, was precipitated.

To a dried flask, the intermediate obtained above and tetrachloromethane were added. The mixture was cooled to -10 °C or below with stirring, and then triethylamine was added thereto to give a reaction solution. After the solution becoming clear, sulfuryl chloride in tetrachloromethane solution was added dropwise at a very slow rate such that the solution was kept at -10°C or below and was stirred for 10 minutes to 2 hours. Subsequently, the temperature of the solution was slowly raised to ambient temperature. The resultant mixture was cooled to -10°C or below, and water was added to the mixture dropwise. After stirring, an aqueous layer was separated from the mixture. The aqueous layer was dried, and tetrachloromethane was added to dissolve the obtained matter. To the solution, thionyl chloride was added with stirring. The obtained solution was warmed to 40-80°C, and triethylamine was added dropwise to the solution. The solution was stirred at 40-80°C, and then was slowly cooled to 0°C. The obtained solution was washed successively with water, saturated sodium bicarbonate, and water for 1 to 5 times, and an organic layer was separated. The organic layer was dried over anhydrous sodium sulfate, and the dried organic layer was cooled to -10 °C or below, and then Material 2 was added thereto. Ammonia was introduced slowly, and the pH of the obtained solution was adjusted to be greater than 9. The resultant solution was stirred. The solvent in the solution was removed under reduced pressure, and after removing of the solvent, concentrated hydrochloric acid was added to the residue. The resultant was stirred at about 40°C. After completion, the reaction mixture was cooled to 0°C in an ice bath, and a saturated sodium bicarbonate solution was added to adjust the pH to about 5, so that a solid was precipitated. The obtained solid was recrystallized from anhydrous ethanol to give a compound of the formula (II).

### Step 2 Preparation of the compound of the formula (IV)

To a dried flask, a solution of a compound of formula (III') in acetonitrile was added and cooled below -10°C. Then diisopropylethylamine and a solution of a compound (II) in acetonitrile were added to the resultant mixture, and the reaction mixture was stirred. After the reaction finished, ethyl acetate was added to dilute the reaction mixture and the mixture was washed successively with water and saturated brine. The organic layer was then dried and concentrated to give the compound of formula (IV).

### Step 3 Preparation of the compound of the formula (V)

The compound of the formula (IV) was dissolved in a mixture of THF and water, and 10% Lindlar Pd-C was added. The temperature of the mixture was elevated, and was stirred at a hydrogen pressure of 2MPa. Pd-C was removed by filtration and ethyl acetate was added to the filtrate. After layering, an aqueous layer was collected. An aqueous solution was added to the organic layer. The resultant was kept still, and the aqueous layer is separated. The above operation was repeated, and the obtained aqueous layers were combined. Ethanol was slowly added dropwise to the combined aqueous layers at 0°C. The mixture was then cooled to -10°C and stirred at -10°C, and filtrated. The filter cake was recrystallized from acetone to give the compound of the formula (V).

R², R³, R⁴, 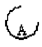 and L in the aforesaid reaction scheme are as defined above. According to the chemical or clinical needs, the compounds of the formulae (IV) and (V) can be further prepared into a pharmaceutically acceptable salt or hydrolysable ester.

In addition, another embodiment of the present invention is a compound of the formula (II), or salts, hydrolysable esters or isomers thereof, 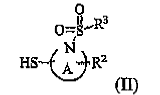 wherein R², R³ and 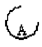 are as defined above. Examples of the specific compounds (II) are the intermediates obtained in Step 3 of the Preparation Examples 1 to 5 corresponding to the aforesaid compounds 1 to 5, and the intermediates obtained in Step 2 of the Preparation Examples 6 to 10 corresponding to the aforesaid compounds 6 to 10.

The pharmaceutically acceptable salts of any of the above compounds according to the present invention are organic acid salts, inorganic acid salts, organic base or inorganic base salts, wherein the organic acids include acetic acid, trifluoroacetic acid, methanesulfonic acid, toluenesulfonic acid, maleic acid, succinic acid, tartaric acid, citric acid, and fumaric acid; the inorganic acids include hydrochloric acid, hydrobromic acid, nitric acid, sulphuric acid, and phosphoric acid; the organic bases include meglumine and dextrosamine; and the inorganic bases include the alkaline compounds containing sodium, potassium, barium, calcium, magnesium, zinc or lithium. The commonly used pharmaceutically acceptable salts are sodium salts and potassium salts, for example, sodium salt of (4R,5S,6S)-3-[1-(2-hydroxy-3-amino-propylaminosulfonyl)-azetidin-3-yl]thio-6-[( 1R)-1-hydroxyethyl]-4-methyl-7-oxo-1-aza-bicyclo-[3.2.0]hept-2-ene-2-carboxylate acid.

The hydrolysable esters of the compound according to the present invention are those esters that can be hydrolyzed into the corresponding carboxylic acids in a biological body. Examples of the hydrolysable esters formed by the compound with carboxyl group include, for example, lower alkanoyloxyalkyl esters, including isopropylformyloxymethyl esters, tert-butylformyloxymethyl esters, neopentylformylaxymethyl esters, isobutylformyloxymethyl esters, neopentylacetoxymethyl esters, octanoyloxymethyl esters, and decanoyloxymethyl esters; cycloalkanoyloxyalkyl esters, including cyclohexylformyloxymethyl esters, cyclohexylformyloxy-1-ethyl esters, 1-methyl-cyclohexylformyloxy-1-ethyl esters, and 4-methylcyclohexylformyloxymethyl esters; lower alkoxylacyloxyalkyl esters, including ethoxyformyloxymethyl esters, isopropoxyformyloxy-1-ethyl esters, hexyloxyformyloxy-1-ethyl esters, octoxyformyloxy-1-ethyl esters, decanoxyformyloxy-1-ethyl esters, and dodecyloxyformyloxy-1-ethyl esters; cycloalkoxylacyloxyalkyl esters, including cyclopentanoxyformyloxy-1-ethyl esters, and cyclohexyloxyformyloxy-1-ethyl esters; (5-methyl-2-oxo-1,3-dioxol-4-yl) methyl esters and the like; hydrolysable esters formed at the nitrogen atom of pyrrolidine, such as lower alkanoyloxymethoxycarbonyl esters, including propionyloxymethoxycarbonyl esters, butyryloxymethoxycarbonyl esters, tert-butylformyloxymethoxycarbonyl esters, isopropylformyloxymethoxycarbonyl esters, and isopropoxyformyloxy-1-ethoxycarbonyl esters; cycloalkanoyloxymethyloxycarbonyl esters, including cyclohexyloxyformyloxy-1-ethoxycarbonyl esters, (5-methyl-2-oxo-1,3-dioxol-4-yl) methyl esters; allyloxycarbonyl esters. The preferred hydrolysable esters are those esters formed with a carboxylic acid. Examples of such esters include propionyloxymethyl esters, butyryloxymethyl esters, tert-butylformyloxymethyl esters, isopropoxyformyloxymethyl esters, isopropoxyformyloxy-1-ethyl esters, cyclohexyloxyformyloxy-1-ethyl esters, and (5-mothyl-2-oxo-1,3-dioxole-4-yl) methoxycarbonyl esters; esters formed at N atom of pyrrolidine, for example, propionyloxymethoxycarbonyl esters, butyryloxymethoxycarbonyl esters, tert-butylformyloxymethoxycarbonyl esters, isopropoxyformyloxymethoxycarbonyl esters, isopropoxyformyloxy-1-ethoxycarbonyl esters, cyclohexyloxyformyloxy-1-ethoxycarbonyl esters, and (5-methyl-2-oxo-1,3-dioxole-4-yl) methyl esters. Commonly used esters include pivaloyloxymethyl esters, (5-methyl-2-oxo-1,3-dioxole-4-yl)methyl esters, e.g. (4R,5S, 6S)-3-[1-(2-acetamido)aminosulfonyl-azetidin-3-yl]thio-6-[(1R)-1-hydroxyethyl]-4-methyl-7-oxo-1-aza-bicyclo-[3.2.0]hept-2-ene-2-carboxylic acid pivaloyloxymethyl esters.

The term "isomer" as used herein, refers to all epimeric, diastereomeric and tautomeric forms of the compounds according to the present invention. When a bond is drawn as a wedge, this indicates that in three dimensions the bond would be coming out of the paper and when a bond is hatched, this indicates that in three dimensions the bond would be going back into the paper. The compounds of the formula (I) contain a number of chiral centers, including those at the position 4, 5 and/or 6. When the compounds of the present invention contain olefinic double bonds, they have cis- and trans-geometrical isomers.

The compounds of the formula (I), and the pharmaceutically acceptable salts, the hydrolysable esters, and the isomers thereof may be in a hydrate form. The hydration may be accomplished in the preparation process, or gradually performed by using the hygroscopic property of the original anhydrous product.

The present invention further relates to a pharmaceutical composition comprising any of the aforesaid compounds, pharmaceutically acceptable salts thereof, hydrolysable esters thereof, isomers thereof, hydrates thereof, or hydrates of the esters or salts and other pharmaceutically active ingredients. The said other pharmaceutically active ingredients are e.g. cilastatin and sodium salt of cilastatin, and/or betamipron, etc.

Another embodiment of this invention pertains to a pharmaceutical composition comprising any of the aforesaid compounds, pharmaceutically acceptable salts, hydrolysable esters, isomers or hydrates thereof, or hydrates of the said esters or salts and one or more pharmaceutical carrier(s) and/or diluent(s). The composition is in any clinically or pharmaceutically acceptable dosage forms, preferably oral or injectable formations. The unit dose of the composition comprises a compound of the formula (I) in a physiologically effective amount. The amount of a compound of the formula (1) according to the invention in the unit dose of the composition may range from 0.01 to 10 g, *e.g*. 0.01 g, 0.015 g, 0.02 g, 0.025 g, 0.03 g, 0.04 g, 0.05 g, 0.1 g, 0.125 g, 0.2 g, 0.25 g, 0.3 g, 0.4 g, 0.5 g, 0.6 g, 0.75 g, 1 g, 1.25 g, 1.5 g, 1.75 g, 2 g, 2.5 g, 3 g, 4 g, 5 g, 6 g, 7 g, 8 g, 9 g, 10 g *etc*.

Any of the aforesaid compound according to the present invention, pharmaceutically acceptable salts, hydrolysable esters, isomers or hydrates thereof, or hydrates of the said esters or salts can be applied to a patient in need thereof through oral or parenteral routes.

When the pharmaceutical composition is administered through parenteral routes, it can be formulated into an injectable dosage form. The term "an injectable dosage form" as used herein, refers to the sterile formulation made of the compound and being injectable, which comprises solutions, emulsions or suspensions, or the sterile powders or concentrated solutions for reconstitution or dilution into sterile injectable solutions or suspensions immediately before use. The injectable dosage form can be classified into an injectable liquid, a sterile powder for injection, and a concentrated solution for injection. The term "injectable liquid" as used herein, refers to a sterile solution-type for injection, emulsion for injection or suspension for injection made of the compound, which can be administrated intramuscularly, intravenously, infusion and etc. The dose strengths of the injections may be 1 ml, 2 ml, 5 ml, 10 ml, 20 ml, 50 ml, 100 ml, 200 ml, 250 ml, 500 ml and etc., of which the large volume (usually not less than 100ml) injection for intravenous infusion is also known as an intravenous transfuse. The term "sterile powder for injection" as used herein, refers to the sterile powder or clumpy substance made of the compound for reconstituting into an injectable solution or homogeneous suspension with a suitable sterile solution immediately before use. It can be used as injection after being reconstituted with suitable solvent for injection, as intravenous infusion after being reconstituted with intravenous transfusion. The sterile powder can be prepared by means of crystallization with solvent, spray drying or freeze-drying methods. The term "concentrated solution for injection" as used herein, means the sterile concentrated solution made of the compound, which can be diluted for intravenous infusion just prior to use.

The injectable dosage form can be produced by the conventional methods in the art of formulations, and aqueous solvents or non-aqueous solvents may be selected as the solvents for the injectable dosage forms. The most commonly used aqueous solvent is water for injection, as well as 0.9% sodium chloride solution or other suitable aqueous solutions. The commonly used non-aqueous solvent is vegetable oil, mainly soy bean oil for injection, and others aqueous solutions of alcohol, propylene glycol, polyethylene glycol, and *etc*., During the preparation of an injectable dosage form, additives may not be used or may be used, and suitable additives may also be added in accordance with the nature of the the compound, such as osmotic regulators, pH regulators, solubilizers, fillers, antioxidants, antibacterial agents, emulsifiers, suspending agents, and so on, Commonly used osmotic regulators include sodium chloride, glucose, potassium chloride, magnesium chloride, calcium chloride, sorbitol, etc., preferably sodium chloride or glucose. Commonly used pH regulators include acetic acid-sodium acetate, lactic acid, citric acid-sodium citrate, sodium bicarbonate-sodium carbonate, etc. Commonly used solubilizers include polysorbate 80, propylene glycol, lecithin, polyoxyethylenated castor oil, *etc*.. Commonly used fillers include lactose, mannitol, sorbitol, dextran, etc. Commonly used antioxidants include sodium sulfite, sodium bisulfite, sodium pyrosulfite, etc. Commonly used antibacterial agents include phenol, cresol, trichlorobutanol, etc. Commonly used containers for injection include glass ampoules, glass bottles, plastic ampoules, plastic bottles, etc.

When the pharmaceutical composition according to the present invention is administered orally, it can be formulated into common solid dosage forms for oral administration, for example, tablets, capsules, pills, granules, and so on. It also can be formulated into liquid dosage forms for oral administration, such as oral solutions, oral suspensions, syrups and the like. The term "tablets" as used herein, refers to those solid preparations which are prepared by homogeneously mixing and pressing the compound and suitable excipients into circular or irregular troches, mainly in common tablets for oral administration, including also buccal tablets, sublingual tablets, buccal wafer, chewable tablets, dispersible tablets, soluble tablets, effervescent tablets, sustained-release tablets, controlled-release tablets, enteric-coated tablets and the like. The term "capsules" as used herein, refers to those solid preparations which are prepared by Siting the compound, or the compound together with suitable excipients into hollow capsules or sealing into soft capsule materials. According to the solubility and release property, capsules can be divided into hard capsules (regular capsules), soft capsules (soft shell capsules), sustained-release capsules, controlled-release capsules, enteric-coated capsules and the like. The term "pills" as used herein, refers to spherical or near-spherical solid preparations which are prepared by mixing the compound and suitable excipients via suitable methods, including dropping pills, dragee, pilule and the like. The term "granules" as used herein, refers to dry granular preparations which are prepared by mixing the compound and suitable excipients and have a certain particle size. Granules can be divided into soluble granules (generally referred to as granules), suspension granules, effervescent granules, enteric-coated granules, sustained-release granules, controlled-release granules and the like. The term "oral solutions" as used herein, refers to a settled liquid preparation which is prepared by dissolving the compound in suitable solvents for oral administration. The term "oral suspensions" as used herein, refers to suspensions for oral administration, which are prepared by dispersing insoluble solid medicaments in liquid vehicles, also including dry suspension or concentrated suspension. The term "syrups" as used herein, refers to a concentrated sucrose aqueous solution containing the compound.

Suitable bulking agents, adhesives, disintegrants, lubricants and the like can be used for the preparation of the oral solid dosage forms of the pharmaceutical composition according to the invention. Commonly used bulking agents include starch, sugar powder, calcium phosphate, calcium sulfate dihydrate, dextrin, microcrystalline cellulose, lactose, pregelatinized starch, mannitol and the like. Commonly used adhesives include sodium carboxymethylcellulose, PVP-K30, hydroxypropyl cellulose, starch slurry, methylcellulose, ethylcellulose, hydroxypropyl methyl cellulose, gelling starch and the like. Commonly used disintegrants include dry starch, crospovidone, croscarmellose, sodium carboxymethyl starch, low-substituted hydroxypropyl cellulose and the like. Commonly used lubricants include magnesium stearate, talc, sodium dodecyl sulfate, micronized silica gel and the like.

The present invention further provides the use of a sulfonyl-substituted carbapenem of the formula (1), or pharmaceutically acceptable salts, hydrolysable esters or isomers thereof in the manufacture of a medicament for the treatment and/or prophylaxis of infectious diseases. Said infectious diseases include, for example, 1) respiratory infections, such as chronic bronchitis, pneumonia, pulmonary abscess, and pyothorax; 2) intra-abdominal infections, such as cholecystitis, cholangeitis, liver abscess, and peritonitis; 3) urogenital infections, such as pyelonephritis, complicated cystitis, adnexitis, intra-uterine infection, pelvic inflammation, and parametritis; 4) bone, arthrosis, cutaneous and parenchyma infections, such as cellulitis, perianal abscess, osteomyelitis, arthritis, trauma wound infection, burn wound infection, operative incision infection, and inflammation in jaw bone and areolar tissue around jaw bone; 5) ocular and ENT infection; and 6) further severe infections, such as meningitis and septicemia.

The sulfonyl-substituted carbapenem of the formula (1) according to the present invention has a good antibacterial activity against gram positive and negative, and aerobic and anaerobic bacteria, and exhibits a low toxicity. It is useful for the treatment and/or prophylaxis of various diseases caused by pathogenic microorganisms. The compound of the present invention has a strong affinity to Pups, a broad antibacterial spectrum, a high antibacterial activity, and a good antibacterial activity against gram positive and negative, aerobic and anaerobic bacteria and pathogens causing nosocomial infections. Especially, it has a prominent antibacterial activity against drug-resistant gram positive and negative bacteria. It is stable to beta-lactamases and Dizzy, and can be administered independently as a single drug. It has a long post antibiotic effect and an enduring antibacterial effect, so that the administration times can be reduced.

It has been discovered that the carbapenem antibiotics usually are nontoxic to warm blood animals. And this discovery is applicable to the compound of the present invention. When the compound of present invention is administered to mice in an amount which is excessive over the dose required for the prophylaxis of bacterial infections, no distinct sign of toxicity or side-effect induced by the compounds of the present invention is observed.

### Examples

The present invention will be further illustrated with the following examples, but the protection scope of the present invention is not limited to the examples. Any variations and modifications obvious to one skilled in the art are intended to be covered by the scope of the present invention. The excipients used in the following examples for preparation of the compositions may be replaced by pharmaceutically acceptable excipients, or may be reduced or increased.

### Preparation examples of the compounds according to the present invention

### Example 1

### Preparation of (4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxylic acid (compound 1)

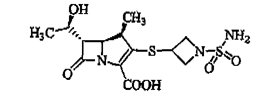

### Step 1 Preparation of 3-acetylthio-azetidine

To a dried flask, 6.9 g (40 mmol) of 3-hydroxy-1-tert-butoxycarbonyl-azetidine (purchased from Wuhan Shenlong Science & Technology Co. Ltd.) and 9.2 g (80 mmol) of sodium thioacetate was added, and then 100 mL of DMF was added thereto. The resulting mixture was heated with being stirred until dissolution. The obtained mixture was kept warm for 12 hours, and then was cooled to room temperature. 100 mL of water was added to dilute the mixture. The resulting mixture was extracted with ethyl acetate. The organic phase was washed with water, and then dried and concentrated to give a yellow oily matter. 50 mL of dichloromethane was added to the obtained oily matter, and, at ambient temperature, 20 mL of trifluoroacetic acid (TFA) was added dropwise thereto. The obtained mixture was stirred over night. TLC was used to monitor whether the reactants therein had been fully reacted. The resultant was concentrated, 50 mL of dichloromethane was added to dilute the concentrate, and the resultant was rotary-eyaporated to give the residue. The above operations were repeated to remove the residual TFA. To the obtained residue 50 mL of methanol was added, and 4.3 g of a light yellow solid was precipitated with a yield of 81.5%,

### Step 2 Preparation of 3-mercapto-N-aminosulfonyl-azetidine

To a dried flask, 13.1 g (100 mmol) of 3-acetylthio-azetidine obtained in the above step and 100 mL of tetrachloromethane were added. The resultant was cooled to -20°C with being stirred, and then 11.1 g (110 mmol) of triethylamine was added thereto to give a solution. After the solution becoming clear, 12.8 g (110 mmol) of sulfuryl chloride in tetrachloromethane was added dropwise at a very slow rate. The obtained mixture was stirred for 0.5 hours at -20°C. Subsequently, the temperature of the solution was slowly raised to ambient temperature. After stirring the resultant for 0.5 hours, the reaction was terminated. The resultant mixture was cooled to a temperature below -10°C, and 50 mL water was added into the mixture dropwise. After stirring, an aqueous layer was separated. The aqueous layer is a solution of 3-acetylthio-N-sulfonyl-azetidine. It was dried, and kept for use.

To a dried flask, a tetrachloromethane solution of the 3-acetylthio-N-sulfonyl-azetidine obtained above was added. 11.9 g (100 mmol) of thionyl chloride was added under stirring. The obtained mixture was warmed to 60°C, and 15.2 g (150 mmol) of triethylamine was slowly added thereto dropwise. The solution was kept warm under stirring for 0.5 hours. Subsequently, the solution was slowly cooled to 0°C. The obtained solution was washed successively with water, saturated sodium bicarbonate, and water, and the organic layer was collected and dried over anhydrous sodium sulfate, and then was cooled to a temperature below -10°C. To the resultant, dry ammonia gas was introduced slowly until the pH of the mixture was greater than 9. The organic layer was stirred for 0.5 hours. The solvent in the solution was removed under reduced pressure, and 50 mL of concentrated hydrochloric acid was added to the residue. The resultant was stirred at 0°C for 1 hour in an ice bath, a saturated sodium bicarbonate solution was added to adjust the pH of the mixture to about 5, so that a solid was precipitated. The obtained solid was recrystallized from anhydrous ethanol, and 12.1 g of 3-mercapto-N-aminosulfonyl-azetidine was obtained with a yield of 72.1 %.

### Step 3 Preparation of p-nitrobenzyl (4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-en e-2-carboxylate

To a dried flask, a solution of 30g (50mmol) of p-nitrobenzyl (4R,5S,6S)-3-diphenoxyphosphoryloxy-6-[(1R)-1-hydroxyethyl]-4-mothyl-7-oxo-1-aza-bicycl o[3.2.0]hept-2-ene-2-carboxylate (purchased from Xinxiang Hongchen Science & Technology Co. Ltd.) in 200 mL of acetonitrile was added and cooled below -10°C. Then 10 mL of diisopropylethylamine and a solution of 8.6 g (51 mmol) of 3-mercapto-N-aminosulfonyl-azetidine obtained above in 100 mL of acetonitrile were added to the resultant mixture, and the reaction mixture was stirred for 15 hours at 0°C. After the stirring is finished, 300 mL of ethyl acetate was added to dilute the reaction mixture, and the mixture was washed successively with water and saturated brine. The organic layer was collected, dried, and concentrated to give a solid of 13.9 g with a yield of 54.4%.

### Step 4 Preparation of the compound 1

20.5 g (40 mmol) of p-nitrobenzyl (4R,5S,6S)-3-[N-aminosulfonyl-azetidin-3-yl]thio-6-[(1R)-1-hydroxyethyl]-4-mehyl-7-oxo-1-aza-bicyclo-[3.2.0]hept-2-en e-2-carboxylate obtained above was dissolved in a mixture of 200 mL of THF and 40 mL of water, and 5 g of 10% Lindlar Pd-C (purchased from Shanghai Hufeng Biological Science & Technology Co. Ltd.) was added. The temperature of the mixture was elevated to 40°C, and the mixture was stirred for 2 hours under a hydrogen pressure of 2MPa. The Pd-C was removed by filtration and 50 mL of ethyl acetate was added to the filtrate. After layering, an aqueous layer was collected. 20 mL of an aqueous solution was added to the organic layer. The resultant was kept still, and the aqueous layer is separated. The above operation was repeated once, and the obtained aqueous layers were combined. 200 mL of ethanol was slowly added deopwise to the combined aqueous layers at 0°C. The mixture was then cooled to -10°C and stirred for 1 hour, and filtered. The filtered cake was recrystallized from acetone to give 8.0 g of the title compound with a yield of 53.1%.

Formula: C₁₃H₁₉N₃O₆S₂

Molecular weight: 377.44
Element analysis:
Found: C, 41.23%; H, 5.38%; N, 11.02%; S, 16.75%
Cacd.: C, 41.37%; H, 5.07%; N, 11.13%; S, 16.99%
Mass spectrum: m/e: 378 (M+1)
¹H-NMR: δ1.16 (d, 3H), 1,21 (d, 3H), 2.00 (s, 3H), 2.96 (m, 1H), 3.14 (t, 1H), 3.27 (m, 1H), 3.42 (t, 1H), 3.61 (m, 1H), 4.05 (d, 1H), 4.07 (d, 1H), 4.31 (d, 1H), 4.32 (d, 1H), 11.0 (s, 1H).

### Example 2

### Preparation of (4R,5S,6S)-3-[(3S)-N-aminosunlfonyl-pyrrolidin-3-yl]-thio-6-[(1R)-1-hydroxyethyl]-4-methyl-7-oxo-1-aza-bicyclo-[3,2,0]hept-2-ene-2-carboxylic acid (compound 2)

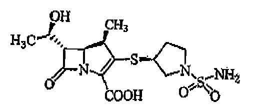

### Step 1 Preparation of (3S)-3-acetylthio-pyrrolidine

The preparation was conducted according to the preparation method in the step 1 of Example 1, except for the replacement of 3-hydroxy-1-tert-butoxycarbonylazetidine with 7.5 g (40 mmol) of (3S)-3-hydroxy-1-tert-butoxycarbonylpyrrolidine, to give 4.7 g of the product with a yield of 80.7%.

### Step 2 Preparation of (3S)-3-mercapto-N-aminosulfonyl-pyrrolidine

The preparation was conducted according to step 2 of Example 1, except for the replacement of 3-acetylthio-azetidine with 14.5 g (100 mmol) of the aforesaid (3S)-3-acetylthio-pyrrolidine, to give 12.8 g of the product with a yield of 70.5%.

### Step 3. Preparation of p-nitrobenzyl (4R,5S,6S)-3-[(3S)-N-aminosulfonylpyrrolidin-3-yl]-thio-6-[(1R)-1-hydroxyethyl]-4-methyl-7-oxo-1-aza-bicyclo-[3.2.0 ]hept-2-ene-2-carboxylate

The preparation was conducted according to step 3 of Example 1, and 30 g (50mmol) of p-nitrobenayl (4R,5S,6S)-3-diphenoxyphosphoryloxy-6-[(1R)-1-hydroxyethyl]-4-methyl-7-oxo-1-aza-bicyclo[3.2.0]hept-2-ene-2-carboxylate was used, except for the replacement of 3-mercapto-N-aminosulfonyl-azetidine with 9.1 g (51 mmol) of (3S)-3-mercapto-N-aminosulfonyl-pyrrolidine, to give 4.5 g of product with a yield of 55.2%.

### Step 4 Preparation of the compound 2

The preparation was conducted according to step 4 of Example 1, except for the replacement of p-nitrobenzyl (4R,5S,6S)-3-[N-aminosulfonyl-azetidin-3-yl]-thio-6-[(1R)-1-hydroxyethyl]-4-methyl-7-oxo-1-aza-bicyclo-[3.2.0]hept-2-ene-2-c arboxylate with 21.1g (40mmol) of the p-nitrobenzyl (4R,5S,6S)-3-[(3S)-N-aminosulfonyl-pyrrolidin-3-yl]thio-6-[(1R)-1-hydroxyethyl]-4-methyl-7-oxo-1-aza-bicyclo-[3.2.0]hept-2-ene-2-carboxylate prepared in the aforesaid step 3, to give 7.6g of the product with a yield of 48.6%.

Formula: C₁₄H₂₁N₃O₆S₂

Molecular weight: 391.46
Element analysis:
Found: C, 42.74%; H, 5.68%; N, 10.53%; S, 16.12%
Cacd.: C, 42.95%; H, 5.41 %; N, 10.73%; S, 16.38%
Mass spectrum: m/e: 392 (M+1)
¹H-NMR: δ1.17 (d, 3H), 1.23 (d, 3H), 2.05 (s, 3H), 2.10 (m, 1H), 2.35 (m, 1H), 2.63 (m, 1H), 2.70 (m, 1H), 2.81 (t, 1H), 2.96 (m, 1H), 3.14 (t, 1H), 3.31 (m, 1H), 3.42 (t, 1H), 3.53 (m, 1H), 3.61 (m, 1H), 11.3 (s, 1H).

### Example 3

### Preparation of (4R,5S,6S)-3-[N-aminosulfonyl-piperidin-3-yl]thio-6-[(1R)1-hydroxyethyl)-4-methyl-7-oxo-1-aza-bicyclo-[3.2.0]hept-2-ene-2-carboxylic acid (compound 3)

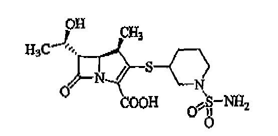

### Step 1 Preparation of 3-acetylthio-niperidine

The preparation was conducted according to step 1 of Example 1, except for the replacement of 3-hydroxy-1-tert-butoxycarbonyl-azetidine with 8.1 g (40 mmol) of 3-hydroxy-1-tert-butoxycarbonyl-piperidine, to give 4.9 g of the product with a yield of 77.2%.

### Step 2 Preparation of 3-mercapto-N-aminosulfonyl-piperidine

The preparation was conducted according to step 2 of Example 1, except for the replacement of 3-acetylthio-azetidine with 16 g (100 mmol) of 3-acetylthio-piperidine, to give 14.5 g of the product with a yield of 74.3%.

### Step_3 Preparation of p-nitrobenzyl_(4R,5S,6S)-3-[N-aminosulfonyl-1-piperidin-3-yl]thio-6-[(1R)-1-hydroxyethyl]-4-methyl-7-oxo-1-aza-bicyclo-[3.2.0]hept-2-ene-2-carboxylate

The preparation was conducted according to step 3 of Example I, and 30 g (50 mmol) of p-nitrobenzyl (4R,5S,6S)-3-diphenoxyphosphoryloxy-6-[(1R)-1-hydroxyethyl]-4-methyl-7-oxo-1-aza-bicyclo[3.2.0]hept-2-ene-2-carboxylate was used, except for the replacement of 3-mercapto-N-aminosulfonyl-azetidine with 9.8 g (51 mmol) of 3-mercapto-N-aminosulfonyl-piperidine, to give 13.7 g of the product with a yield of 50.8%.

### Step 4 Preparation of the compound 3

The preparation was conducted according to step 4 of Example 1, except for the replacement of p-nitrobenzyl (4R,5S,6S)-3-[N-aminosulfonyl-azetidin-3-yl]-thio-6-[(1R)-1-hydroxyethyl]-4-methyl-7-oxo-1-aza-bicyclo-[3.2.0]hept-2-ene-2-carboxylate with 21.6 g (40 mmol) of the p-nitrobenzyl (4R,5S,6S)-3-[N-aminosulfonyl-piperidin-3-yl]thio-6-[(1R)-1-hydroxyethyl]-4-methyl-7-oxo-1-aza-bicyclo -[3.2.0]hept-2-ene-2-carboxylate prepared in the aforesaid step 3, to give 8.1 g of the product with a yield of 49.8%.

Formula: C₁₅H₂₃N₃O₆S₂

Molecular weight: 405.49
Element analysis:
Found : C, 44.21%; H, 5.94%; N, 10.41%; S, 15.65%
Cacd.: C, 44.43%; H, 5.72%; N, 10.36%; S, 15.82%
Mass spectrum: m/e: 406 (M+1)
¹H-NMR: δ1.15 (d, 3H), 1.22 (d, 3H), 1.45 (m, 1H), 1.55 (m, 1H), 2.00 (s, 3H), 2.01 (t, 1H), 2.26 (m, 1H), 2.52 (m, 1H), 2.68 (m, 1H), 2.80 (t, 1H), 2.95 (m, 1H), 3.16 (t, 1H), 3.20 (m, 1H), 3.44 (t, 1H), 3.51 (m, 1H), 3.63 (m, 1H), 11.2 (s, 1H).

### Example 4

### Preparation of (4R,5S,6S)-3-[1(4H)-aminosulfonyl-5,6-dihydropyrimidin-5-yl]thio-6-[(1R)-1-hydroxyethyl]-4-methyl-7-oxo-1-aza-bicyclo-[3.2.0]hept-2-ene-2-carboxylic acid (compound 4)

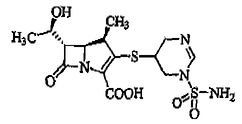

### Step 1 Preparation of 5-acetylthio-1,4,5,6-tetrahydropyrimidine

The preparation was conducted according to step 1 of Example 1, except for the replacement of 3-hydroxy-1-tert-butoxycarbonyl-azetidine with 8.0 g (40 mmol) of 5-hydroxy-1-tert-butoxycarbonyl-1,4,5,6-tetrahydropyrimidine, to give 4.8 g of the product with a yield of 75.1 %.

### Step 2 Preparation of 5-mercapto-1(4H)-aminosulfonyl-5,6-dihydropyrimidine

The preparation was conducted according to step 2 of Example 1, except for the replacement of 3-acetylthio-azetidine with 15.8 g (100 mmol) of 5-acetylthio-1,4,5,6-tetrahydropyrimidine, to give 5.1 g of the product with a yield of 65.4%.

### Step 3 Preparation of p-nitrobenzyl (4R,5S,6S)-3-[1(4H)-aminosulfonyl-5,6-di-hydropyrimidin-5-yl]thio-6-[(1R)-1-hydroxyethyl]-4-methyl-7-oxo-1-aza-bicyclo-[3.2.0]hept-2-ene-2-carboxylate

The preparation was conducted according to step 3 of Example 1, and 30 g (50 mmol) p-nitrobenzyl (4R,5S,6S)-3-diphenoxyphosphoryloxy-6-[(1R)-1-hydroxyethyl]-4-methyl-7-oxo-1-aza-bicyclo[3.2.0]hept-2-ene-2-carboxylate was used, except for the replacement of 3-mercapto-N-aminosulfonyl-azetidine with 10 g (51 mmol) of 5-mercapto-1(4*H*)-aminosulfonyl-5,6-dihydropyrimidine, to give 13.1 g of the product with a yield of 48.6%.

### Step 4 Preparation of the compound 4

The preparation was conducted according to step 4 of Example 1, except for the replacement of p-nitrobenzyl (4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxylate with 21.6 g (40 mmol) of the p-nitrobenzyl (4R,5S,6S)-3-[1(4*H*)-aminosulfonyl-5,6-dihydropyrimidin-5-yl]thio-6-[(1R)-1-hydroxyethyl]-4-methyl-7-oxo-1-aza-bicyclo-[3.2.0]hept-2-ene-2-carboxylate prepared in the aforesaid step 3, to give 7.5 g of the product with a yield of 46.6%.

Formula: C₁₆H₂₀N₄O₆S₂

Molecular weight: 404.46
Element analysis:
Found : C, 41.36%; H, 5.16%; N, 13.73%; S, 15.75%
Cacd.: C, 41.57%; H, 4.98%; N, 13.85%; S, 15.86%
Mass spectrum: m/e: 405 (M+1)
¹H-NMR: δ1,16 (d, 3H), 1.20 (d, 3H), 1.80 (q, 1H), 2.00 (s, 3H), 2.11 (q, 1H), 2.81 (m, 1H), 2.96 (m, 1H), 3.13 (d, 1H), 3.16 (d, 1H), 3.42 (t, 1H), 3.44 (t, 1H), 3.61 (m, 1H), 7.50 (s, 1H), 11.2 (s, 1H).

### Example 5

### Preparation of (4R,5S,6S)-3-[1(2H)-aminosulfonyl-tetrahydropyrimidin-5-yl]-thio-6-[(1R)-1-hydroxyethyl]-4-methyl-7-oxo-1-aza-bicyclo-[3.2.0]hept-2-ene-2-carboxylic acid (compound 5)

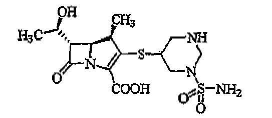

### Step 1 Preparation of 5-acetylthio-1(2H)-tetrahydropyrimidine

The preparation was conducted according to step 1 of Example 1, except for the replacement of 3-hydroxy-1-tert-butoxycarbonyl-azetidine with 12.1 g (40 mmol) of 5-hydroxy-1,3-bis(tert-butoxycarbonyl)-1(2*H*)-hexahydropyrimidine, to give 4.5 g of the product with a yield of 69.5%.

### Step 2 Preparation of 5-mercapto-1(2H)-aminosulfonyl-tetrahydropyrimidine

The preparation was conducted according to step 2 of Example 1, except for the replacement of 3-acetylthio-azetidine with 16 g (100 mmol) of 5-acetylthio-1(2*H*)-tetrahydropyrimidine, to give 12.7 g of the product with a yield of 64.5%.

### Step 3 Preparation of p-nitrobenzyl (4R,5S,6S)-3-[1(2H)-aminosulfonyl-tetrahydropyrimidin-5-yl]thio-6-[(1R)-1-hydroxyethyl]-4-methyl-7-oxo-1-aza-bicyclo-[3.2.0]hept-2-ene-2-carboxylate

The preparation was conducted according to step 3 of Example 1, and 30 g (50 mmol) of p-nitrobenzyl (4R,5S,6S)-3-diphenoxyphosphoryloxy-6-[(1R)-1-hydroxyethyl]-4-methyl-7-oxo-1-aza-bicyclo[3.2.0]hept-2-ene-2-carboxylate was used, except for the replacement of 3-mercapto-N-aminosulfonyl-azetidine with 10,1 g (51 mmol) of 5-mereapto-1(2*H*)-aminosulfonyl-tetrahydropyrimidine, to give 12.5 g of the product with a yield of 46.3%.

### Step 4 Preparation of the compound 5

The preparation was conducted according to step 4 of Example 1, except for the replacement of p-nitrobenzyl (4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxylate with 21.7 g (40 mmol) of the p-nitrobenzyl (4R,5S,6S)-3-[1(2*H*)-aminosulfonyl-tetrahydropyrimidin-5-yl)thio-6-[(1R)-1-hydroxyethyl]-4-methyl-7-oxo-1 -aza-bicyclo-[3,2,0]hept-2-ene-2-carboxylate prepared in the aforesaid step 3, to give 7.4 g of the product with a yield of 45.7%.

Formula: C₁₄H₂₂N₄O₆S₂

Molecular weight: 406.48
Element analysis:
Found: C, 41.22%; H, 5.78%; N, 13.72%; S, 15.56%
Cacd.: C, 41.37%; H, 5.46%; N, 13.78%; S, 15.78%
Mass spectrum: m/e: 407 (M+1)
¹H-NMR: δ1.15 (d, 3H), 1.19 (d, 3H), 2.01 (s, 4H), 2.96 (m, 1H), 3.01 (t, 1H), 3.07 (m, 2H), 3.14 (t, 1H), 3.31 (m, 2H), 3.42 (t, 1H), 3.62 (m, 1H), 3.79 (s, 1H), 3.84 (t, 1H), 11.05 (s, 1H).

### Example 6

### Preparation of (4R,5S,6S)-3-[1-(N,N-dimethyl-aminosulfonyl)-azetidin-3-yl]-thio-6-[(1R)-1-hydroxyethyl]-4-methyl-7-oxo-1-aza-bicyclo-[3.2.0]hept-2-ene-2-carboxylic acid (compound 6)

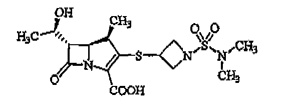

### Step 1 Preparation of 1-(N,N-dimethyl-aminosulfonyl)-3-mercapto-azetidine

The preparation was conducted according to step 2 of Example 1 except for the replacement of triethylamine with 4.7 g (105 mmol) of dimethylamine, to give 12.1 g of the product with a yield of 61.8%.

### Step 2 Preparation of p-nitrobenzyl (4R,5S,6S)-3-[1-(N,N-dimethylaminosulfonyl)-azetidin-3-yl]thio-6-[(1R)-1-hydroxyethyl]-4-methyl-7-oxo-1-aza-bicyclo-[ 3.2.0]hept-2-ene-2-carboxylate

The preparation was conducted according to step 3 of Example 1, and 30 g (50 mmol) of p-nitrobenzyl (4R,5S,6S)-3-diphenoxyphosphoryloxy-6-[(1R)-1-hydroxyethyl]-4-methyl-7-oxo-1-aza-bicyclo[3.2.0]hept-2-ene-2-carboxylate was used, except for the replacement of 3-mercapto-N-aminosulfonyl-azetidine with 10.0 g (51 mmol) of 3-mercapto-1-(N,N-dimethylaminosulfonyl)-azetidine, to give 14.1 g of the product with a yield of 52.1%.

### Step 3 Preparation of the compound 6

The preparation was conducted according to step 4 of Example 1, except for the replacement of p-nitrobenzyl (4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxylate with 21.7 g (40 mmol) of the p-nitrobenzyl (4R,5S,6S)-3-[1-(N,N-dimethylaminosulfonyl)-azetidin-3-yl]thio-6-[(1R)-1-hydroxyethyl]-4-methyl-7-oxo-1-aza-bicyclo-[3.2.0]hept-2-ene-2-carboxylate prepared in the aforesaid step 2, to give 8.3g of the product with a yield of 51.2%.

Formula: C₁₅H₂₃N₃O₆S₂

Molecular weight: 405.49
Element analysis:
Found: C, 44.25%; H, 5.89%; N, 10.15%; S, 15.97%
Cacd.: C, 44.43%; H, 5.72%; N, 10.36%; S, 15.82%
Mass spectrum: m/e: 406 (M+1)
¹H-NMR: δ1.14 (d, 3H), 1.22 (d, 3H), 2.02 (s, 1H), 2.47 (s, 6H), 2.96 (m, 1H), 3.14 (t, 1H), 3.27 (m, 1H), 3.41 (t, 1H), 3.62 (m, 1H), 4.06 (d, 1H), 4.09 (d, 1H), 4.30 (d, 1H), 4.32 (d, 1H), 11.05 (s, 1H).

### Example 7

### Preparation of (4R,5S,6S)-3-[1-(N,N-diethyl-aminosulfonyl)-azetidin-3-yl]thio-6-[(1R)-1-hydroxyethyl]-4-methyl-7-oxo-1-aza-bicyclo-[3.2.0]hept-2-ene-2-carboxylic acid (compound 7)

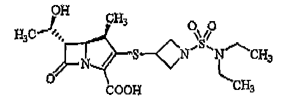

### Step 1 Preparation of 1-(N,N-diethylaminosulfonyl)-3-mercapto-azetidine

The preparation was conducted according to step 2 of Example 1, except for the replacement of triethylamine with 7.7 g (105 mmol) of diethylamine, to give 13.8 g of the product with a yield of 61.3%.

### Step 2 Preparation of p-nitrobenzyl (4R,5S,6S)-3-[1-(N,N-diethylaminosulfonyl)-azetidin-3-yl]thio-6-[(1R)-1-hydroxyethyl]-4-methyl-7-oxo-1-aza-bicyclo-[3.2.0]hept-2-ene-2-carboxylate

The preparation was conducted according to step 3 of Example 1, and 30 g (50 mmol) of p-nitrobenzyl (4R,5S,6S)-3-diphenoxyphosphoryloxy-6-[(1R)-1-hydtoxyethyl]-4-methyl-7-oxo-1-aza-bicyclo[3.2.0]hept-2-ene-2-carboxylate was used, except for the replacement of 3-mercapto-N-aminosulfonyl-azetidine with 11.4 g (51 mmol) of 3-mercapto-1-(N,N-diethylaminosulfonyl)-azetidine, to give 14.6 g of the product with a yield of 51.4%.

### Step 3 Preparation of the compound 7

The preparation was conducted according to step 4 of Example I, except for the replacement of p-nitrobenzyl (4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxy late with 22.7 g (40 mmol) of the p-nitrobenzyl (4R,5S,6S)-3-[1-(N,N-diethylaminosulfonyl)-azetidin-3-yl]thio-6-[(1R)-1-hydroxyethyl]-4-methyl-7-oxo -1-aza-bicyclo-[3.2.0]hept-2-ene-2-carboxylate prepared in the aforesaid step 2, to give 8.6 g of the product with a yield of 49.8%.

Formula: C₁₇H₂₇N₃O₆S₂

Molecular weight: 433.54
Element analysis:
Found : C, 46.89%; H, 5.54%; N, 9.43%; S, 14.96%
Cacd.: C, 47.10%; H, 6.28%; N, 9.69%; S, 14.79%
Mass spectrum: m/e; 434 (M+1)
¹H-NMR: δ1.03 (t, 6H), 1.15 (d, 3H), 1.20 (d, 3H), 2.03 (s, 1H), 2.59 (q, 4H), 2.98 (m, 1H), 3.17 (t, 1H), 3.28 (m, 1H), 3.42 (t, 1H), 3.61 (m, 1H), 4.05 (d, 1H), 4.08 (d, 1H), 4.31 (d, 1H), 4.33 (d, 1H), 11.00 (s, 1H).

### Example 8

### Preparation of (4R,5S,6S)-3-[1-(N,N-dibutyl-aminosulfonyl)-azetidin-3-yl]-thio-6-[(1R)-1-hydroxyethyl]-4-methyl-7-oxo-1-aza-bicyclo-[3.2.0]hept-2-ene-2- carboxylic acid (compound 8)

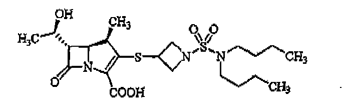

### Step 1 Preparation of 1-(N,N-dibutylaminosulfonyl)-3-mercapto-azetidine

The preparation was conducted according to step 2 of Example 1, except for the replacement of triethylamine with 13.6 g (105 mmol) of dibutylamine, to give 16.7 g of the product with a yield of 59.7%.

### Step 2 Preparation of p-nitrobenzyl (4R,5S,6S)-3-[1-(N,N-dibutylaminosulfonyl)-azetidin-3-yl]thio-6-[(1R)-1-hydroxyethyl]-4-methyl-7-oxo-1-aza-bicyclo-[ 3.2.0]hept-2-ene-2-carboxylate

The preparation was conducted according to step 3 of Example 1, and 30 g (50 mmol) of p-nitrobenzyl (4R,5S,6S)-3-diphenoxyphosphoryloxy-6-[(1R)-1-hydroxyethyl]-4-methyl-7-oxo-1-aza-bicyclo[3.2.0]hept-2-one-2-carboxylate was used, except for the replacement of 3-mercapto-N-aminosulfonyl-azetidine with 14.3 g (51 mmol) of 3-mercapto-1-(N,N-dibutylaminosulfonyl-azetidine, to give 15.1 g of the product with a yield of 48.3%.

### Step Preparation of the compound 8

The preparation was conducted according to step 4 of Example 1, except for the replacement of p-nitrobenzyl (4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carbo xylate with 25.0 g (40 mmol) of the p-nitrobenzyl (4R,5S,6S)-3-[1-(N,N-dibutylaminosulfonyl)-azetidin-3-yl]thio-6-[(1R)-1-hydroxyethyl]-4-methyl-7-oxo -1-aza-bicyclo-[3.2.0]hept-2-ene-2-carboxylate prepared in the aforesaid step 2, to give 9.4 g of the product with a yield of 48.2%.

Formula: C₂₁H₃₅N₃O₆S₂

Molecular weight: 489.65
Element analysis:
Found: C,51.23%; H,7.56%; N,8.35%; S,13.29%
Cacd.: C,51.51%; H,7.20%; N,8.58%; S,13.10%
Mass spectrum: m/e: 490 (M+1)
¹H-NMR: δ0.95 (t, 6H), 1.16 (d, 3H), 1.22 (d, 3H), 1.33 (q, 4H), 2.03 (s, 1H), 2.55 (m, 4H), 2.59 (q, 4H), 2.98 (m, 1H), 3.17 (t, 1H), 3.28 (m, 1H), 3.42 (t, 1H), 3.61 (m, 1H), 4.05 (d, 1H), 4.08 (d, 1H), 4.30 (d, 1H), 4.33 (d, 1H), 11.02 (s, 1H).

### Example 9

### Preparation of (4R,5S,6S)-3-[1-(2-hydroxy-3-amino-propylaminosulfonyl)-azetidin-3-yl]thio-6-[(1R)-1-hydroxyethyl]-4-methyl-7-oxo-1-aza-bicyclo-[3.2.0]hept-2-ene-2-carboxylic acid (compound 9)

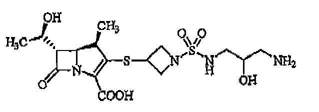

### Step 1 Preparation of 1-(2-hydroxy-3-aminopropylaminosulfonyl)-3-mercaptoazetidine

The preparation was conducted according to step 2 of Example 1, except for the replacement of triethylamine with 9.5 g (105 mmol) of 2-hydroxy-1,3-propylenediamine, to give 12.9 g of the product with a yield of 53.6%.

### Step 2 Preparation of p-nitrobenzyl (4R,5S,6S)-3-[1-(2-hydroxy-3-aminopropylaminosulfonyl)-azetidin-3-yl]thio-6-[(1R)-1-hydroxyethyl]-4-methyl-7-oxo-1-aza-bicyclo-[3.2.0]hept-2-ene2-carboxylate

The preparation was conducted according to step 3 of Example 1, and 30 g (50 mmol) of p-nitrobenzyl (4R,5S,6S)-3-diphenoxyphosphoryloxy-6-[(1R)-1-hydroxyethyl]-4-methyl-7-oxo-1-aza-bicyclo[3.2.0]hept-2-ene-2-carboxylate was used, except for the replacement of 3-mercapto-N-aminosulfonyl-azetidine with 12.3 g (51 mmol) of 3-mercapto-1-(2-hydroxy-3-amino-propylaminosulfonyl)-azetidine, to give 13.3 g of the product with a yield of 45.5%.

### Step 3 Preparation of the compound 9

The preparation was conducted according to step 4 of Example 1, except for the replacement of p-nitrobenzyl (4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxy late with 23.4 g (40 mmol) of the p-nitrobenzyl (4R,5S,6S)-3-[1-(2-hydroxy-3-amino-propylaminosulfonyl)-azetidin-3-yl]thio-6-[(1R)-1-hydroxyethyl ]-4-methyl-7-oxo-1-aza-bicyclo-[3.2.0]hept-2-ene-2-carboxylate prepared in the aforesaid step 2, to give 8.4 g of the product with a yield of 46.6%.

Formula: C₁₆H₂₆N₄O₇S₂

Molecular weight: 450.53
Element analysis:
Found : C,42.33%; H,6.02%; N,12.15%; S,14.47%
Cacd.: C,42.65%; H,5.82%; N,12.44%; S,14.23%
Mass spectrum: m/e: 451 (M+1)
¹H-NMR: δ1.14 (d, 3H), 1.20 (d, 3H), 2.03 (s, 5H), 2.67 (q, 2H), 2.92 (m, 2H), 2.97 (m, 1H), 3.15 (t, 1H), 3.26 (m, 1H), 3.44 (t, 1H), 3.60 (m, 1H), 3.69 (m, 1H), 4.04 (d, 1H), 4.07 (d, 1H), 4.29 (d, 1H), 4.32 (d, 1H), 11.01 (s, 1H),

### Example 10

### Preparation of (4R,5S,6S)-3-[1-(2-acetamido-aminosulfonyl)-azetidin-3-yl] thio-6-[(1R)-1-hydroxyethyl]-4-methyl-7-oxo-1-aza-bicyclo-[3.2.0]hept-2-ene-2- carboxylic acid (compound 10)

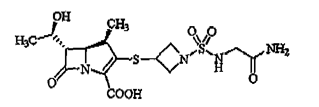

### Step 1 Preparation of 1-(N,N-dimethyl-aminosulfouyl)-3-mercapto-azetidin

The preparation was conducted according to step 2 of Example 1, except for the replacement of triethylamine with 7.8 g (105 mmol) of 2-amino-acetamide, to give 12.5 g of the product with a yield of 55.6%.

### Step 2 Preparation of p-nitrobenzyl (4R,5S,6S)-3-[1-(2-acetamido-aminosulfonyl)-azetidin-3-yl]thio-6-[(1R)-1-hydroxyethyl]-4-methyl-7-oxo-1-aza-bicyclo-[3.2.0]hept-2-ene-2-carboxylate

The preparation was conducted according to step 3 of Example 1, and 30 g (50 mmol) of p-nitrobenzyl (4R,5S,6S)-3-diphenoxyphosphoryloxy-6-[(1R)-1-hydroxyethyl]-4-methyl-7-oxo-1-aza-bicyclo[3.2.0]hept-2-ene-2-carboxylate was used, except for the replacement of 3-mercapto-N-aminosulfonyl-azetidine with 11.3 g (51 mmol) of 3-mercapto-1-(2-acetamido-aminosulfonyl)-azetidine, to give 13.4 g of the product with a yield of 47.1 %.

### Step 3 Preparation of the compound 10

The preparation was conducted according to step 4 of Example 1, except for the replacement of p-nitrobenzyl (4R,5S,65)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxylate with 22.8 g (40 mmol) of the p-nitrobenzyl (4R,5S,65)-3-[1-(2-acetamidoaminosulfonyl)-azetidin-3-yl]thio-6-[(1R)-1-hydroxyethyl]-4-methyl-7-oxo-1-aza-bicyclo-[3.2.0]hept-2-ene-2-carboxylate prepared in the aforesaid step 2, to give 7.9 g of the product with a yield of 45.2%.

Formula: C₁₅H₂₂N₄O₇S₂

Molecular weight: 434.49
Element analysis:
Found : C,41.22%; H,5.38%; N,12.67%; S,14.85%
Cacd.: C,41.47%;H,5.10%; N,12.89%; S,14.76%
Mass spectrum: m/e: 435 (M+1)
¹H-NMR: δ1.16 (d, 3H), 1.21 (d, 3H), 2.00 (s, 2H), 2.96 (m, 1H), 3.14 (t, 1H), 3.27 (m, 1H), 3.42 (t, 1H), 3.54 (s, 2H), 3.61 (m, 1H), 4.05 (d, 1H), 4.07 (d, 1H), 4.31 (d, 1H), 4.32 (d, 1H), 6.02 (s, 2H), 11.0 (s, 1H).

### Example 11

### Preparation of pivaloyloxymethyl (4R,5S,6S)-3-[1-(2-acetamido-aminosulfonyl)-azetidin-3-yl]thio-6-[(1R)-1-hydroxyethyl]-4-methyl-7-oxo-1-aza-bicyclo-[3.2.0]hept-2-ene-2-carboxylate (pivaloyloxymethyl ester of the compound 10)

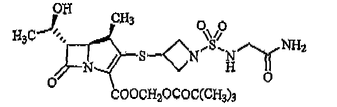

2.2 g (5 mmol) of (4R,5S,6S)-3-[1-(2-acetamido)aminosulfonyl-azetidin-3-yl]-thio-6-[(1R)-1-hydroxyethyl]-4-methyl-7-oxo-1-aza-bicyclo-[3.2.0]hept-2-ene-2-carboxylic acid (the compound 10 obtained in Example 10) was added to 35 mL of N,N-dimethylformamide, The resulting mixture was cooled to 0°C, and 1 g of triethylamine was added thereto. The mixture was stirred untill dissolution, and 1.3 g (5.5 mmol) of iodomethyl pivalate was added and stirred for 1 hour. After completion of the reaction, the resultant was poured into a mixture of 150 mL of water and 150 mL of ethyl acetate; and sodium bicarbonate was added to adjust the pH to about 7.0. The resultant was filtered, and the organic layer was separated, dried over anhydrous magnesium sulfate. The organic layer was rotary-dried, and the residue was dissolved in ethyl acetate, and cooled in an ice bath, an ether solution containing HCl was added dropwise into the resultant. The precipitate formed was collected by filtration, and recrystallized from chloroform to give a white solid of 1.5 g with a yield of 54.2%.

### Example 12

### Preparation of sodium (4R,5S,6S)-3-[1-(2-hydroxy-3-amino-propylaminosulfonyl-azetidin-3-yl]thio-6-[(1R)-1-hydroxyethyl]-4-methyl-7-oxo-1-aza-bicyclo-[3.2.0]hept-2-ene-2-carboxylate (sodium salt of the compound 9)

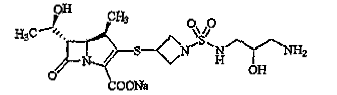

4.5 g (10 mmol) of (4R,5S,6S)-3-[1-(2-hydroxy-3-aminopropylaminosulfonyl)-azetidin-3-yl]thio-6-[(1R)-1-hydroxyethyl]-4-methyl-7-oxo-1-aza-bicyclo-[3.2.0]-hept-2-ene-2-carboxylic acid (the compound 9 obtained in Example 9) was dissolved in 20 mL of deionized water, stirred, and cooled to about 0°C. 0.85 g powdery sodium bicarbonate was slowly added, and the resultant was stirred at 0 °C for 2 hours. The resultant was then filtered through 0.2 µm microporous filtration membrane. The filtrate was freeze-dried, and the solid was collected and sufficiently washed with anhydrous ethanol. The obtained solid was vacuum-dried at ambient temperature for 24 hours to give a sodium salt of 3.4 g with a yield of 72.3%.

### Preparation examples of the formulations according to the present invention

### Formulation Example 1

### Preparation of sterile powders for injection of the compounds according to the present invention

### 1. Formula:

### Formula 1

| | |
|---|---|
| Compound 1 | 10 g |
| arginine | 990 g |
| Units prepared | 1000 bottles |

### Formula 2

| | |
|---|---|
| Compound 2 | 500 g |
| dextran | 500 g |
| Units prepared | 1000 bottles |

### Formula 3

| | |
|---|---|
| Sodium salt of | 1000 g (calcd, on the |
| compound 9 | basis of the compound) |
| Units prepared | 1000 bottles |

### Formula 4:

| | |
|---|---|
| Compound 6 | 10 g |
| arginine | 990 g |
| Units prepared | 1000 bottles |

### Formula 5

| | |
|---|---|
| Compound 7 | 500 g |
| dextran | 500 g |
| Units prepared | 1000 bottles |

### Formula 6

| | |
|---|---|
| Compound 8 | 100 g |
| arginine | 900 g |
| Units prepared | 1000 bottles |

### 2. Procedure:

The glass bottles and rubber stoppers used for the formulations were sterilized. In accordance with the formulae above-mentioned, the compounds (being fed after conversion) and excipients were weighted, The resultant was filled in a racking machine with measuring the amounts of the loads at any moment. The bottles were stopped and capped. The finished products were sampled for full tests, packaged and warehoused.

### Formulation Example 2

### Preparation of lyophilized powders of the compounds according to the present invention

### 1. Formula

### Formula 1

| | |
|---|---|
| Compound 3 | 500 g |
| Mannitol | 300 g |
| Water for injection | Suitable amount |
| Units prepared | 1000 bottles |

### Formula 2

| | |
|---|---|
| Compound 4 | 1000 g |
| dextran | 500 g |
| Sodium hydroxide | 20 g |
| Water for injection | Suitable amount |
| Units prepared | 1000 bottles |

2. Procedure: To a mixture of the active ingredients and the excipients in the aforesaid formulae, about 40-80% water for injection was added, so as to dissolve the mixture. The water for injection was supplemented as balance. Activated charcoal for injection was then added in an amount of 0.01-0.1% of the mixing liquid to adsorb the resultant for 15-30 min. The resultant was filtered to remove the activated charcoal, and then was fine-filtered. The semifinished products were obtained, and the contents of the compounds according to the invention in the semifinished products were tested, Subsequently, the semifinished products were lyophilized The lyophilizing process comprises the following steps: pre-freezing the semifinished products at -35 to -45°C for 2 to 5 hours, elevating the temperature to 0°C by 1 to 2 degrees every one hour to perform a low-temperature vacuum-drying, and rapidly heating the resultant to 20-40°C to perform a elevated temperature vacuum-drying. The vacuum degree during the above drying process was controlled to be below 0.1mm Hg.

### Formulation Example 3

### Preparation of tablets of the compounds of the present invention

### 1. Formula

### Formula 1

| | |
|---|---|
| Isopropoxycarbonyloxyethyl ester of compound 10 | 250 g (calcd. on the basis of the compound) |
| Pre-gelatinized starch | 80 g |
| Microcrystalline cellulose | 50 g |
| 1% HPMC aqueous solution | Suitable amount |
| Micronized silica gel | 4.0 g |
| Magnesium stearate | 4.0g g |
| Units prepared | 1000 tablets |

### Formula 2

| | |
|---|---|
| Compound 5 | 125 g |
| Starch | 100 g |
| Low-substituted hydroxypropyl cellulose | 50 g |
| Microcrystalline cellulose | 4.0 g |
| 2% PVP (K 30) aqueous solution | Suitable amount |
| Micronized silica gel | 4.0 g |
| Units prepared | 1000 tablets |

### 2. Procedure:

The compounds and excipients were weighted separately according to the proportions in the formulae above for the following operations. The compounds of the present invention were milled and sieved (100 mesh), and the excipients were respectively sieved (100mesh). The compounds of the invention, pre-gelatinized starch (or starch) and microcrystalline cellulose (or low-substituted hydroxypropyl cellulose) were homogeneously mixed, and the resultant was fed to a mixing-granulator. A suitable amount of 1%HPMC aqueous solution (or 2% PVP (K 30) aqueous solution) was then added, and the mixture was stirred for 15 min to form granules. The resulting granules were dried at the temperature below 60 °C. The dried granules were then mixed with micronized silica gel (and magnesium stearate), and then passed through sieves and were homogeneously mixed to obtain the semifinished products. Sampling was performed to test the semifinished products. Tabletting was performed according to the tablet weight determined on the basis of the test. The finished products were sampled for full tests, packaged and warehoused.

### Antibacterial activity of the compound of the invention

**Bacterial strains for test:** All the bacterial strains are clinical isolates and purchased from public organizations. methicillin-sensitive staphylococcus aureus (MSSA), methicillin-resistant staphylococcus aureus (MRSA), methicillin-sensitive staphylococcus epidermidis (MSSE), methicillin-resistant staphylococcus epidermidis (MRSE), streptococcus pyogenes, penicillin-sensitive streptococcus pneumoniae (PSSP), penicillin-resistant streptococcus pneumoniae (PRSP), Escherichia coli, Citrobacter freundii, Enterobacter cloacae, Hemophilus influenza, Klebsiella pneumonia (producing ESBL), Morganella morganii, Proteus mirabilis, Serratia marcescens, and Pseudomonas aeruginosa.

**Drugs to be tested:** compounds of the invention prepared in the examples; the chemical names, structural formulae, preparation materials, and preparation methods are provided in the preparation examples of the compounds;

Meropenem (Meropenem for injection), Ertapenem (Ertapenem for injection), and Doripenem (Doripenem for injection) commercially available

**Experimental method:** the agar dilution method, by reference to Methodology of Pharmacological Experiment (XU Shuyun et al, published by the Peoples Medical Publishing House, 1st Edition, August, 1982; 3rd Edition, 5th printing, January 2002, p1659-1660).

### Experimental results and conclusion:

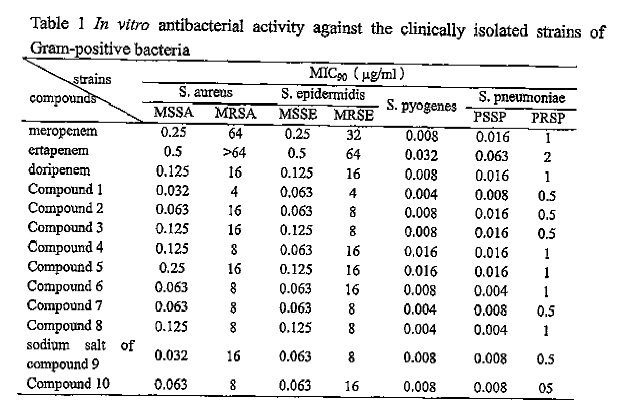

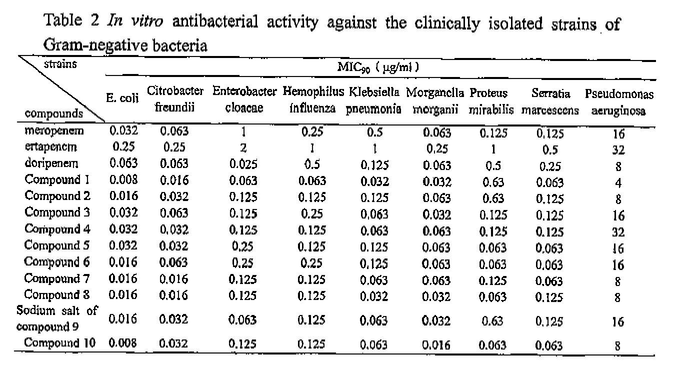

Conclusion: the test results in Tables 1 and 2 showed that the compounds of the present invention had a good antibacterial activity against the clinically isolated strains of Gram-positive and/or Gram-negative bacteria, as compared to meropenem, doripenem and ertapenem. This indicated that the compounds of the invention had a strong antibacterial effect on Gram-positive bacteria, Gram-negative bacteria, and their drug-resistant bacteria in clinic. Accordingly, it is deduced that the compounds have a very strong affinity to penicillin-binding proteins (PBPs). Compared to the closest prior art, the compounds of the invention have an equivalent or better antibacterial activity and have a broad antibacterial spectrum. They have good potential in clinical application.

### Pharmacokinetic study of the compounds according to the invention

Pharmacokinetic study of the compounds in SD rat

### Drugs to be tested and preparation thereof

Test drugs: the compound 1, 6, 8 and 9 prepared in the examples

Control drugs: Meropenem (Meropenem for injection), commercially available; Doripenem (Doripenem for Injection).

Internal standard substance: Warfarin, a white powder, purity 99%, batch number: 0072-8501, provided by Shanghai Institute for Drug Control.

Drug formulation: the test drugs were formulated before use by being dissolved in physiological saline to make a final concentration of 5 mg/mL for intravenous injection.

Test animal: SD male rats, weight: 200~250 g, purchased from SHANGHAI SLAC LABORATORY ANIMAL CO. LTD.

### Animal Experiment:

Drug administration: The SD male rats were randomly divided into five groups with 3 in each group, and given the drugs by intravenous injection at a dose of 10 mg/kg. The animals were weighed before administration.

Collection of Samples: Zero time was marked before administration; 0.5 mL of blood was respectively sampled from orbit venous plexus at 5 min, 15 min, 30 min, 45 min, 1 h, 2 h, 4 h, 6 h, 8 h, 24 h after administration into heparinized centrifuge tubes. The samples were centrifuged for 6 minutes at 8000rpm and the supernatant plasma was separated. The plasma was frozen at -20 °C for LC-MS/MS analysis.

### Development of liquid chromatography-mass spectrometry (LC-MS) method:

Chromatographic conditions: column: Gemini C6-Phenyl (50 mm×4.6 mm, 5 µm); mobile phase: 0.1% of formic acid-water-acetonitrile (5:35:60, v/v/v); flow rate: 1mL/min; column temperature: 35-40°C; injection volume: 5 µL; split ratio: 1/5.

Mass spectrometer conditions: scanning mode: cation multiple reactions monitoring (MRM); ion source: electrospray ionization (ESI); Nebulize gas: 8 L/min; Curtain gas: 8 L/min; Collision gas: 4 L/min; Ionspray voltage: 4500v; Temperature: 400°C/500°C.

Preparation of the standard curve and quality-control sample: a suitable amount of the test drug was weighed precisely and then formulated into a stock solution (2.6mg/mL) with ultrapure water. The stock solution was diluted with methanol to obtain a series of working solutions in a concentration of 25000, 5000, 2500, 500, 250 and 50 ng/mL, respectively. 100 µL of plasma samples were separately added into 20 µL of said working solutions to obtain the calibration solutions in a concentration of 5000, 1000, 500, 100, 50 and 10 ng/mL. By the same method, the quality-controlled sample solutions in a concentration of 4000, 800 and 20 ng/mL can be prepared. A standard curve was obtained by chromatographic analysis of the samples.

Sample preparation: 20 µL of acetonitrile and 200 µL (200 ng/mL) of Warfarin in acetonitrile were added into 100 µL of plasma sample, then spun for 1 minute and centrifuged for 5 minutes at 15000 rpm. 100 µL of the supernatant was taken, where 3 µL aliquot of the supernatant was used for the LC/MS/MS analysis.

### Results and discussions:

Dosing concentration: The accuracy of the drug concentration in dosing solution for intravenous injection was 103.2%, determined by analysis of the formulated drugs using HPLC and comparison with the standards.

Data analysis: The drug concentration in plasma would be considered as zero if under the detection limit (10 ng/mL). The pharmacokinetics parameters were calculated by the non-compartment model using Winnonlin Professional 5.2 pharmacokinetics software.

Pharmacokinetics: The pharmacokinetics parameters and drug concentration-time curve were obtained by measuring the plasma drug concentration at different time points. The half lives (t_{1/2}) of the tested compounds were summarized in Table 3, which showed that the half lives of the compounds of the present invention in rats (intravenous injection) were longer than those of Meropenem and Doripenem.

**Table 3 Half lives of a part of the compounds of the present invention (n=3)**

| Pharmacokinetic data | h ( X±SD) | | | | | |
|---|---|---|---|---|---|---|
| | Meropenem | Doripenem | Compound 1 | Compound 6 | Compound 8 | Compound 9 |
| t_{1/2} | 0.12±0.01 | 0.11±0.01 | 0.32±0.02 | 0.30±0.03 | 0.27±0.03 | 0.32±0.06 |

Since the compounds of the invention have notably increased half lives as compared to those of Meropenem and Doripenem, the compounds have a longer post antibiotic effect and a more enduring antibacterial effect. Thus, the administration times of the drug can be reduced.

## Claims

1. A compound of the formula (I), or pharmaceutically acceptable salts, hydrolysable esters, isomers or hydrates thereof, or hydrates of the said esters or salts: 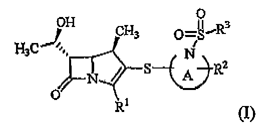 wherein
R¹ represents a carboxyl group, -COOR⁴ or a hydrolysable ester, said R⁴ representing a carboxyl protecting group;
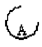 represents a saturated or unsaturated 3- to 7-membered heterocycle containing 1 to 2 nitrogen atoms;
R² represents a hydrogen atom, a halogen atom, hydroxy, amino, carboxy, cyano, nitro, trifluoromethyl, a lower alkyl group, or a lower alkoxy group; and
R³ represents hydroxy or -NR⁵R⁶, where each of R⁵ and R⁶ independently represents a hydrogen atom or a lower alkyl group, said lower alkyl group being optionally substituted with one or more substituents selected from the group consisting of hydroxy, amino, amido, aminosulfonyl, a halogen atom, carboxy, cyano, a lower alkoxy group, trifluoromethoxy, difluoromethoxy, trifluoromethyl, and a combination thereof.

2. A compound according to Claim 1, wherein the compound of the formula (I) has the formula (I'): 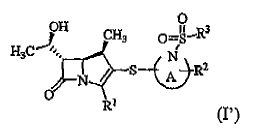 wherein
R¹ represents a carboxyl group, -COOR⁴ or a hydrolysable ester, said R⁴ representing a carboxyl protecting group;
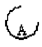 represents a saturated or unsaturated 3- to 7-membered heterocycle containing 1 to 2 nitrogen atoms;
R² represents a hydrogen atom, a halogen atom, hydroxy, amino, carboxy, cyano, nitro, trifluoromethyl, a lower alkyl group, or a lower alkoxy group; and
R³ represents hydroxy or -NR⁵R⁶, where each of R⁵ and R⁶ independently represents a hydrogen atom or a lower alkyl group, said lower alkyl group being optionally substituted with one or more substituents selected from the group consisting of hydroxy, amino, amido, aminosulfonyl, a halogen atom, carboxy, cyano, a lower alkoxy group, trifluoromethoxy, difluoromethoxy, trifluoromethyl, and a combination thereof.

3. A compound according to Claim 1, or pharmaceutically acceptable salts, hydrolysable esters, isomers or hydrates thereof, or hydrates of the said esters or salts, wherein
R¹ represents a carboxyl group, -COOR⁴ or a hydrolysable ester, said R⁴ representing a carboxyl protecting group selected from the group consisting of methyl, methoxymethyl, methylthiomethyl, benzoxymethyl, benzoylmethyl, ethyl, tert-butyl, allyl, benzyl, and p-nitrobenzyl, and said hydrolysable ester being selected from the group consisting of lower alkanoyloxyalkyl esters, cycloalkanoyloxyalkyl esters, lower alkoxylacyloxyalkyl esters, cycloalkoxylacyloxyalkyl esters, and (5-methyl-2-oxo-1,3-dioxol-4-yl) methyl esters;
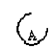 represents a saturated or unsaturated 4- to 6-membered heterocycle containing 1 to 2 nitrogen atoms, which is selected from the group consisting of azetidine, 1,2-diazetidine, 1,2-dihydroazetine, 1,2-dihydro-1,2-diazetine, pyrrolidine, pyrrole, dihydropyrrole, pyrazole, pyrazolidine, imidazole, azacyclohexane, 5,6-dihydropyrimidine, tetrahydropyrimidine, piperidine, and piperazine;
R² represents a hydrogen atom, a fluorine atom, hydroxy, amino, carboxy, methyl, trifluoromethyl, ethyl, methoxy, or ethoxy; and
R³ represents -NR⁵R⁶, where each of R⁵ and R⁶ independently represents a hydrogen atom or a substituted or unsubstituted C₁-C₆ alkyl.

4. A compound according to Claim 3, or pharmaceutically acceptable salts, hydrolysable esters, isomers or hydrates thereof, or hydrates of the said esters or salts, wherein
R¹ represents a carboxyl group, -COOR⁴ or a hydrolysable ester, said R⁴ representing a carboxyl protecting group selected from the group consisting of methyl, methoxymethyl, methylthiomethyl, benzoxymethyl, benzoylmethyl, ethyl, tert-butyl, allyl, benzyl, and p-nitrobenzyl, and said hydrolysable ester being selected from the group consisting of lower alkanoyloxyalkyl esters, cycloalkanoyloxyalkyl esters, lower alkoxyacyloxyalkyl esters, cycloalkoxyacyloxyalkyl esters, and (5-methyl-2-oxo-1,3-dioxol-4-yl) methyl esters;
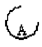 is selected from the group consisting of azetidine, pyrrolidine, piperidine, 5,6-dihydropyrimidine, and tetrahydropyrimidine;
R² represents a hydrogen atom; and
R³ represents hydroxy or -NR⁵R⁶, where each of R⁵ and R⁶ independently represents a hydrogen atom or represents methyl, ethyl, propyl, isopropyl, butyl or isobutyl unsubstituted or substituted by the substituents selected from the group consisting of hydroxy, amino, amido, and a combination thereof.

5. A compound according to Claim 4, or pharmaceutically acceptable salts, hydrolysable esters, isomers or hydrates thereof, or hydrates of the said esters or salts, wherein
R¹ represents a carboxyl group, -COOR⁴ or a hydrolysable ester, said R⁴ representing a carboxyl protecting group selected from the group consisting of methyl, allyl and benzyl, and said hydrolysable ester being selected from the group consisting of propionyloxymethyl esters, butyryloxymethyl esters, tert-butylformyloxymethyl esters, isopropoxyformyloxymethyl esters isopropoxyformyloxy-1-ethyl esters, cyclohexyloxyformyloxy-1-ethyl esters and (5-methyl-2-oxo-1,3-dioxol-4-yl) methyl esters;
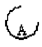 is selected from the group consisting of azetidine, pyrrolidine, piperidine, 5,6-dihydropyrimidine, and tetrahydropyrimidine;
R² represents a hydrogen atom; and
R³ represents -NH₂.

6. A compound according to Claim 1, or pharmaceutically acceptable salts, hydrolysable esters, isomers or hydrates thereof, or hydrates of the said esters or salts, said compound being selected from the group consisting of:
(4R,5S,6S)-3-[N-aminosulfonyl-azetidin-3-yl]thio-6-[(1R)-1-hydroxyethyl]-4-methyl-7-oxo-1-aza-bicyclo-[3.2.0]hept-2-ene-2-carboxylic acid;
(4R,5S,6S)-3-[(3S)-N-aminosulfonyl-pyrrolidin-3-yl]thio-6-[(1R)-1-hydroxy ethyl]-4-methyl-7-oxo-1-aza-bicyclo-[3.2.0]hept-2-ene-2-carboxylic acid;
(4R,5S,6S)-3-[N-aminosulfony-piperidin-3-yl]-thio-6-[(1R)-1-hydroxyethyl] -4-methyl-7-oxo-1-aza-bicyclo-[3.2.0]hept-2-ene-2- carboxylic acid;
(4R,5S,6S)-3-[1(4*H*)-aminosulfonyl-5,6-dihydropyrimidin-5-yl]-thio-6-[(1R) -1-hydroxyethyl]-4-methyl-7-oxo-1-aza-bicyclo-[3.2.0]hept-2-ene-2-carboxylic acid;
(4R,5S,6S)-3-[1(2*H*)-aminosulfonyl-tetrahydropyrimidin-5-yl]-thio-6-[(1R)-1-hydroxyethyl]-4-methyl-7-oxo-1-aza-bicyclo-[3.2.0]hept-2-ene-2-carboxylic acid;
(4R,5S,6S)-3-[1-(N,N-dimethylaminosulfonyl)-azetidin-3-yl]thio-6-[(1R)-1-hydroxyethyl]-4-methyl-7-oxo-1-aza-bicyclo-[3.2.0]hept-2-ene-2-carboxylic acid;
(4R,5S,6S)-3-[1-(N,N-diethylaminosulfonyl)-azetidin-3-yl]thio-6-[(1R)-1-hydroxyethyl]-4-methyl-7-oxo-1-aza-bicyclo-[3.2.0]hept-2-ene-2-carboxylic acid;
(4R,5S,6S)-3-[1-(N,N-dibutylaminosulfonyl)-azetidin-3-yl]thio-6-[(1R)-1-hydroxyethyl]-4-mothyl-7-oxo-1-aza-bicyclo-[3.2.0]hept-2-one-2-carboxylic acid;
(4R,5S,6S)-3-[1-(2-hydroxy-3-aminopropylaminosulfonyl)-azetidin-3-yl]thio -6-[(1R)-1-hydroxyethyl]-4-methyl-7-oxo-1-aza-bicyclo-[3.2.0]hept-2-ene-2-carboxylic acid; and
(4R,5S,6S)-3-[1-(2-acetamido)aminosulfonyl-azetidin-3-yl]-thio-6-[(1R)-1-hydroxyethyl]-4-methyl-7-oxo-1-aza-bicyclo-[3.2.0]hept-2-ene-2-carboxylic acid.

7. A compound according to any one of Claims 1 to 6, or pharmaceutically acceptable salts, hydrolysable esters, isomers or hydrates thereof, or hydrates of the said esters or salts, wherein the pharmaceutically acceptable salts are organic acid salts, inorganic acid salts, organic base or inorganic base salts, wherein said organic acids are selected from acetic acid, trifluoroacetic acid, methanesulfonic acid, toluenesulfonic acid, maleic acid, succinic acid, tartaric acid, citric acid, and fumaric acid; said inorganic acids are selected from hydrochloric acid, hydrobromic acid, nitric acid, sulphuric acid, and phosphoric acid; said organic bases are selected from meglumine and dextrosamine; and said inorganic bases are selected from the alkaline compounds containing sodium, potassium, barium, calcium, magnesium, zinc and lithium.

8. A compound according to any one of Claims 1 to 7, wherein the hydrolysable esters are those esters that can be hydrolyzed into the corresponding carboxylic acids in a biological body.

9. A pharmaceutical composition which comprises a compound according to any one of Claims 1 to 8, or pharmaceutically acceptable salts, hydrolysable esters, isomers or hydrates thereof, or hydrates of the said esters or salts, and one or more pharmaceutically acceptable carriers and/or diluents.

10. A pharmaceutical composition according to Claim 9, which is in any pharmaceutically acceptable dosage form.

11. Use of a compound according to any one of Claims 1 to 8, or pharmaceutically acceptable salts, hydrolysable esters, isomers or hydrates thereof, or hydrates of the said esters or salts in the manufacture of a medicament for the treatment and/or prophylaxis of infectious diseases.

12. A process for preparing a compound of the formula (I), which comprises performing a nucleophilic substitution reaction between a compound of the formula (II) or a salt/ester/isomer thereof with a compound of formula (III), 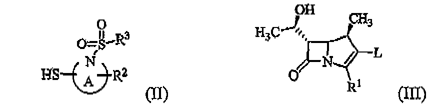 wherein R¹, R², R³ and 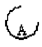 are as defined in Claim 1, and L represents a leaving group.

13. A compound of the formula (II), or a salt, hydrolysable ester or isomer thereof, 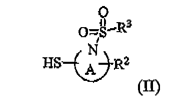 wherein R², R³ and 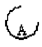 are as defined in Claim 1.
